# EUROPEAN PATENT APPLICATION

(11) **EP 1 880 709 A1**
(43) Date of publication of application: **23.01.2008**
(21) Application number: 06015217.0
(22) Date of filing: 21.07.2006
(51) Int. Cl.: A61K 8/44, A61Q 5/04

(54) **Method and composition for permanently shaping hair**

(71) Applicant: Wella Aktiengesellschaft, 64274 Darmstadt (DE)
(72) Inventor: Cassier, Thorsten, Dr., 64807 Dieburg (DE)

(57) **Abstract**

A composition for permanent hair shaping comprising
(i) 1% to 30 % by weight of an N-alkyl-2-mercaptoacetamide of formula 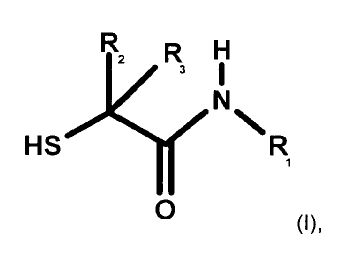 or the salt thereof, wherein R₁ is a straight chain alkyl residue with 3 to 6 carbon atoms or a straight chain hydroxyalkyl residue with 3 to 6 carbon atoms, R₂ and R₃ are, independently from each other, hydrogen or straight chain alkyl residues with 1 to 3 carbon atoms,
(ii) 0.01% to about 20% by weight of at least one at least one cationic surfactant that contains either a quaternary ammonium group or a protonable nitrogen group, and
(iii) 5% to 95% by weight of water;
and process for permanent shaping.

## Description

### Field of the Invention

The object of the present invention is a composition on the basis of a combination of an N-alkyl-2-mercaptoacetamide as a keratin reducing agent and at least one cationic surfactant for permanently shaping hair and a process therefore.

### Prior Art

The classic technique for permanent hair shaping is typically based on two treatment steps: In the first step, the cystine-disulfide bridges of the keratin of the hair are opened by the action of a composition that contains a reducing agent (shaping composition). The hair is then put into the desired shape. In a second step, the cystine-disulfide bonds are closed again using a fixative, that is, a composition comprising an oxidant, usually hydrogen peroxide.

Thioglycolic acid, for instance in the form of its ammonium salt, is used as a classic hair keratin reducing agent but often causes considerable hair damage. Alkaline-adjusted preparations based on mercaptocarboxylic acid esters, despite being adequately effective, cause hair damage, which is expressed for instance in increased hair breakage. Often, these compositions also undesirably stress the scalp. Finally, the unpleasant odor of the conventional reducing agents used, particularly ammonia, requires intensive perfuming of the products. By using 2-mercaptopropionic acid (thiolactic acid), it is possible to solve some of these problems; however, in comparison to thioglycolic acid, which is widely used, thiolactic acid produces weaker waves and the smell is unpleasant as well.

For gentle permanent waving of damaged and especially bleached or dyed hair, waving compositions that are adjusted to be slightly acidic to neutral are preferably desired. From a professional standpoint, over the last 35 years, the thioglycolic acid esters have proved to be the reducing agents best suited for this purpose. A major disadvantage of acidic hair waving compositions based on thioglycolic acid esters, however, is that they are poorly tolerated by the eyes and skin, and the sensitizing effect of the thioglycolic acid esters. As a result the use of these hair waving compositions is widely avoided at present.

In our own EP 0969791 B1 it is reported that N-(3'-hydroxypropyl)-2-mercaptoacetamide has substantially lower sensitization rates than thioglycolic acid and the closest relatives in permanent waving ingredients, the N-alkyl-2-mercapto-acetamides, that is N-methylmercaptoacetamide and N-hydroxyethylmercapto-acetamide, known from the prior art documents DE 1144440 C1 and EP-A 0455457 A1. Never the less by using a N-(3'-hydroxypropyl)-2-mercaptoacetamide as the hair keratine reducing agent the fastness to humidity as particularly sweat still needs improvement.

The goal of the present invention is therefore to provide a composition and a method for permanent shaping hair which improves the fastness to humidity as particularly sweat.

A further goal of the present invention is to provide a permanent shaping composition and method that delivers unique elasticity and strength of the shaped hair from the tip to the root and also an improved uniformity of the shaped hair with less disordered appearance.

Still another goal of the present invention is therefore to provide a composition and a method for permanent shaping hair which improves the odor of the treated hair significantly during and after the hair shaping process and which is superior to former compositions and thus enhances the customers acceptance of permanent hair shaping products.

Still another goal of the present invention is to provide a composition and a method for permanent shaping of hair which leaves hair in a better condition than compositions of the prior art.

Yet another goal of the present invention is to provide a composition for permanent shaping of hair which's skin compatibility is improved compared to compositions of the prior art.

The above problems are solved by a composition and a method as defined in claims 1 and 20 respectively.

The preferred embodiments are indicated in the sub-claims.

The first essential component of the permanent hair shaping composition, the N-alkyl-2-mercaptoacetamide of formula (1), is contained in the composition for permanent shaping hair in a quantity of from about 1 to about 30 % by weight, preferably from about 5 to about 20 % by weight and most preferred from about 8 to about 14 % by weight of the composition.

The preferred salts of N-alkyl-2-mercaptoacetamide of formula (I) are the chloride, the sulfate, the acetate or the citrate. From the N-alkyl-2-mercaptoacetamides of formula (I) the N-(3'-hydroxypropyl)-2-mercaptoacetamide is preferred.

The second essential component of the permanent hair shaping composition is at least one cationic surfactant that contains either a protonable nitrogen group or a quaternary ammonium moiety.

Among the cationic surfactants comprising a quaternary ammonium group those are preferred having the general formula (II)

[NR4,R5,R6,R7]⁺ · X⁻

wherein R₄ to R₇ are independent from each other a saturated or unsaturated aliphatic group of from 1 to 26 carbon atoms or an alkoxy-, polyoxyalkylene-, alkylamido-, hydroxyalkyl-, aminoalkyl-, phenyl-, alkylphenyl-, alkylpyridyl- or benzyl group of from 1 to 26 carbon atoms in the alkyl or alkylene residue; and X⁻ is a salt-forming anion selected from the group consisting of chloride, bromide, iodide; acetate, citrate, lactate, glycolate, phosphate, nitrate, sulfate and methylsulfate.

The aliphatic groups may contain, in addition to carbon and hydrogen atoms, either linkages, and other groups such as amino groups. The longer chain aliphatic groups, e. g. those of about 12 carbons, or higher, can be saturated or unsaturated. Especially preferred are di-long chain (e.g. di C12-C26, preferably C16-C22, aliphatic, preferably alkyl) and di short chain (e.g. di C1 - C3 alkyl, preferably C1 - C2-alkyl) and mono-long chain ammonium salts (e.g. mono C 12-C26, preferably C16-C22, aliphatic, preferably alkyl). Salts of primary, secondary and tertiary fatty amines are also suitable cationic surfactant materials. The alkyl groups of such amines preferably have from about 12 to about 26 carbon atoms, and may be substituted or unsubstituted.

Preferred cationic surfactants, useful herein are selected from the group consisting of (for the nomenclature reference is made to the "International Cosmetic Ingredient Dictionary and Handbook" (CTFA) (8^{th}, 9^{th} and 10^{th} Edition)): C12-C26-alkyl trimethyl ammonium chlorides, preferably cetyl trimethyl ammonium phosphate (CTFA: CETYLDIMONIUM PHOSPHATE), behenyl trimethyl ammonium salts, e. g. behenyl trimethylammonium chloride (CTFA: BEHENTRIMONIUM CHLORIDE) and behenyl trimethylammonium methosulfate (CTFA: BEHENTRIMONIUM METHOSULFAT), stearyl trimethylammonium chloride (CTFA: STEARTRIMONIUM CHLORIDE), octadecyl dimethyl benzyl ammonium chloride (CTFA: STEARALKONIUM CHLORIDE), docosyl dimethyl benzyl ammonium chloride (CTFA: BEHENALKONIUM CHLORIDE), dicetyl dimethyl ammonium chloride (CTFA: DICETYLDIMONIUM CHLORIDE), cetyl trimethyl ammonium salts, preferably cetyl trimethyl ammonium chloride (CTFA: CETRIMONIUM CHLORIDE; sold as Genamin^{®} CTAC 100) or cetyl trimethyl ammonium bromide (CTFA: CETRIMONIUM BROMIDE); dimethyl ditallow ammonium salts; 3-behenoyloxy-2-hydroxypropyl-trimethylammonium chloride (CTFA: BEHENOYL PG-TRIMONIUM CHLORIDE), trimethylaminopropyllaureate (CTFA: LAUROYL PG-TRIMONIUM CHLORIDE), coconut trimethylammonium chloride (CTFA: COCOTRIMONIUM CHLORIDE), N,N,N',N',N'-pentamethyl-N-tallowalkyl-1,3-propane-diammonium dichloride (CTFA: TALLOWDIMONIUM PROPYLTRIMONIUM DICHLORIDE; sold as Duoquad^{®} T-50 by Akzo Nobel), benzyl trimethylammonium chloride (CTFA: BENZALKONIUM CHLORIDE), lauryl trimethylammonium chloride (CTFA: LAURTRIMONIUM CHLORIDE), cetyl dimethyl hydroxyethyl ammonium phosphate (CTFA: HYDROXYETHYL CETYLDIMONIUM PHOSPHATE), dicocos dimethyl ammonium chloride (CTFA: DICOCODIMONIUM CHLORIDE), hydroxycetyl hydroxyethyl dimethyl ammonium chloride (CTFA: HYDROXYCETYL HYDROXYETHYL DIMONIUM CHLORIDE), (2-ethyl-hexyl)-dimethyl-stearyl-ammonium chloride (CTFA: STEARYL ETHYLHEXYLDIMONIUM CHLORIDE), tricetyl monomethyl ammonium chloride (CTFA: TRICETYLMONIUM CHLORIDE), 3-trimethylamino-2-hydroxy-propyl isostearate chloride (CTFA: ISOSTEAROYL PG-TRIMONIUM CHLORIDE), 3-trimethylamino-2-hydroxy-propyl palmitate chloride (CTFA: PALMITOYL PG-TRIMONIUM CHLORIDE), trimethyl-[(3-octadecyloxy)propyl]ammonium chloride (CTFA: STEAROXYPROPYLTRIMONIUM CHLORIDE), stearyl amidopropyl dimethylamine; esterquats as for example carboxymethyl trimethylylammonium chloride hexadecylester (CTFA: CETYL BETAINATE CHLORIDE), diesterquats as for example dipalmitylethyl dimethylammoniumchloride (sold as Armocare^{®} VGH70 by Akzo, Germany) or a mixture of distearoylethyl hydroxyethyl ammonium methosulfate and cetylstearyl alcohol (sold as Dehyquart^{®} F-75 by Henkel, Germany).

Preferred cationic surfactants comprising a protonable nitrogen group are dimethylaminopropyl stearamid (CTFA: STEARAMIDOPROPYL DIMETHYLAMINE), stearyl dimethylamin oxide (CTFA: STEARAMINE OXIDE), dimethylaminopropyl lauramide (CTFA: LAURAMIDOPROPYL DIMETHYLAMINE), N,N-dimethyl-1-behenamine-N-oxide (CTFA: BEHENAMINE OXIDE), N-[3-(dimethylamino)propyl]-cocoamide (CTFA: COCAMIDOPROPYL DIMETHYLAMINE), diethylaminoethyl stearamide, N,N-dimethyl-1-octadecan-amine (CTFA: DIMETHYL STEARAMINE), soyamine, soy dimethylamine (CTFA: DIMETHYL SOYAMINE), myristylamine, tridecylamine, ethylstearylamine, N-tallowpropane diamine, ethoxylated (5 moles EO) stearylamine, N,N-bis(2-hydroxyethyl)-stearylamine, distearyl dimethylammonium chloride (CTFA: DISTEARYLDIMONIUM CHLORIDE), octadecylnitrilio)tri-1,2-ethanediyl)tris hydroxyphosphate (CTFA: QUATERNIUM-52; sold e.g. as Dehyquart^{®} SP by Cognis/Germany), and N-eicosyl-1-docosanamine (CTFA: ARACHIDYL-BEHENYLAMINE; CAS 7268-58-8).

Suitable amine salts include the halogen, acetate, phosphate, nitrate, citrate, lactate, and alkyl sulfate salts.

Also preferred cationic surfactants are secected from the group consisting of the salts polyethylenglycol (5) stearyl ammoniumlactate (CTFA: PEG-5 STEARYL AMMONIUM LACTATE), polyethyleneglycol (15) stearmonium chloride (CTFA: PEG-15 STEARMONIUM CHLORIDE), N-[3-(dimethylamino)propyl]-tetra-decanamide phosphate (CTFA: MYRISTAMIDOPROPYL DIMETHYLAMINE PHOSPHATE), N-(stearoyl colamino formylmethyl)-pyridinium chloride, (CTFA: STEAPYRIUM CHLORIDE; CAS 1341-08-8), 1,3-bis[(N,N-dimethyl-N-stearyl)]-2-hydroxy-propane dichloride (CTFA: HYDROXYPROPYL BISSTEARYLDIMONIUM CHLORIDE), 2-hydroxypropanoic acid compd. with N-(3-(dimethylamino)propyl)-octadecanamide (CTFA: STEARAMIDOPROPYL DIMETHYLAMINE LACTATE), (CTFA: STEARYL ETHYLHEXYLDIMONIUM CHLORIDE), dioleoylethyl hydroxyethyl ammonium methosulfate (CTFA: DIOLEOYLETHYL HYDROXYETHYLMONIUM METHOSULFATE), stearylamine hydrochloride, soyamine chloride, (CTFA: STEARYLAMINE FORMATE), (CTFA: N-TALLOWPROPANE DIAMINE DICHLORIDE) and citric acid compd. with N-(3-(dimethylamino)propyl)-octadecanamide (CTFA: STEARAMIDOPROPYL DIMETHYLAMINE CITRATE).

Most preferred cationic surfactants are cetyl dimethyl hydroxyethyl ammonium phosphate (CTFA: HYDROXYETHYL CETYLDIMONIUM PHOSPHATE), alkyl trimethylammonium chlorides, preferably cetyl trimethyl ammonium phosphate (CTFA: CETYLDIMONIUM PHOSPHATE), behenyl trimethyl ammonium salts, e. g. behenyl trimethylammonium chloride (CTFA: BEHENTRIMONIUM CHLORIDE) and behenyl trimethylammonium methosulfate (CTFA: BEHENTRIMONIUM METHOSULFAT), stearyl trimethylammonium chloride (CTFA: STEARTRIMONIUM CHLORIDE), octadecyl dimethyl benzyl ammonium chloride (CTFA: STEARALKONIUM CHLORIDE), docosyl dimethyl benzyl ammonium chloride (CTFA: BEHENALKONIUM CHLORIDE), dicetyl dimethyl ammonium chloride (CTFA: DICETYLDIMONIUM CHLORIDE), cetyl trimethyl ammonium salts, preferably cetyl trimethyl ammonium chloride (CTFA: CETRIMONIUM CHLORIDE; sold as Genamin^{®} CTAC 100) or cetyl trimethyl ammonium bromide (CTFA: CETRIMONIUM BROMIDE); dimethyl ditallow ammonium salts; stearyl amidopropyl dimethylamine; esterquats as for example (CTFA: TETRADECYL BETAINESTER CHLORIDE), diesterquats as for example dipalmitylethyl dimethylammoniumchloride (sold as Armocare^{®} VGH70 by Akzo, Germany) or a mixture of distearoylethyl hydroxyethyl ammonium methosulfate and cetylstearyl alcohol (sold as Dehyquart^{®} F-75 by Henkel, Germany).

The cationic surfactant is contained in the composition for permanent shaping hair in a quantity of from about 0.01% to about 20% by weight, preferably from about 0.05% to about 8% by weight and most preferred from about 0.1 % to about 3% by weight of the composition.

### Further Hair Keratin Reducing Agent

Although it is preferred that the N-alkyl-2-mercaptoacetamide of formula (I) is the sole keratin reducing agent in the composition of the invention, it is possible that the hair shaping composition of the invention comprises at least one further hair keratin reducing agent selected from the group consisting of thioglycolic acid and its salts, thiolactic acid, 3-mercaptopropionic acid, 2-hydroxy-3-mercaptopropionic acid, cysteine, cysteamine, alkyl- or acylcysteamine with 1 to 4 carbon atoms in the alkyl residue, or the salts thereof; cysteine-(2-hydroxyethyl)ester, L-cysteine glycerine ester and glycerine mono thioglycolate; From these keratin reducing agents, thioglycolic acid, thiolactic acid, cysteine and cysteamine are preferred.

The preferred amount of the further keratin reducing agent is from about 0.5% by weight to about 10% by weight, more preferred from about 1% by weight to about 8% by weight, most preferred from about 1% by weight to about 5% by weight.

The preferred ratio between the hair keratin-reducing agent N-alkyl-2-mercaptoacetamide of formula (I) and the further keratin-reducing agents or the mixture thereof is preferably from about 5 : 1 to about 2:1.

The permanent shaping composition may comprise the keratin-reducing agents, i. e. the mixture of N-alkyl-2-mercaptoacetamide of formula (I) with the other hair keratin reducing agents, particularly thioglycolic acid, thiolactic acid and cysteine or the salts thereof, in a total amount of from about 2 to about 30 %by weight, preferably from about 5 to about 20 % by weight and most preferred from about 6 to about 15 % by weight of the composition. It is further preferred that the other hair keratin-reducing agent be a salt.

### Hair Swelling and Penetration Enhancing Substances

So-called swelling agents and penetration enhancing substances may be added to the hair shaping composition, examples being urea, melamine; ethers, e. g. dipropyleneglycol monomethyl ether or dipropyleneglycol monoethyl ether etc.; 2-pyrrolidon, imidazolidin-2-one, 1-methyl-2-pyrrolidone; alkali or ammonium thiocyanate, polyvalent alcohols, isopropanol. These agents are preferably contained in a quantity of from about 0.1 to about 30 % by weight, more preferred from about 1 to about 10 % by weight of the composition.

### Disulfides

It is further advantageous if the permanent hair shaping composition, in order to avoid making the hair too kinky, contains the disulfide of a hair keratin-reducing compound (thiol), particularly 2,2'-dithio-bis[N-(3-hydroxypropyl)-acetamide], 2,2'-dithio-bis[N-(2-hydroxypropyl)-acetamide], dithioglycolic acid, dithiolactic acid and their salts. The preferred quantity for use is from about 0.1 to about 20 % by weight, more preferably from about 0.5 to about 15 % by weight and most preferred from about 1 to about 10 % by weight of the composition, wherein the ratio between the hair keratin-reducing agents and the disulfides is preferably from about 20 : 1 to about 1 : 2, and particularly from about 10 : 1 to about 1 : 1.

### Additives

It is understood that the hair shaping composition may contain all the usual and known additives for such compositions, such as thickeners and fillers, e. g. kaolin, bentonite, fatty acids, higher fatty alcohols, starches, cellulose derivatives, alginates, xanthan gum, guar gum, polyacrylic acid and its derivatives, cellulose derivatives, alginates, vaseline, and paraffin oils; wetting agents or emulsifiers from the classes of anionic, amphoteric or nonionic surface-active substances, such as fatty alcohol sulfates, fatty alcohol ether sulfates, alkyl sulfonates, alkylbenzene sulfates, alkyl betaines, ethoxylated alkylphenols, fatty acid alkanolamides or ethoxylated fatty acid esters; and opacifiers, such as polyethyleneglycol esters; solvents, such as alcohols, e.g. ethanol, propanol, isopropanol; watersoluble polyhydric alcohols having two or more hydroxyl groups in the molecule. Typical examples of such polyhydric alcohols are dihydric alcohols such as ethylene glycol, 1,2- or 1,3-propanediol, 1,2-pentanediol and glycerine; further, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; pentahydric alcohols such as xylytol, etc.; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerin monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerin ether, POP glycerin ether, POP glycerin ether phosphoric acid, POP POE pentanerythritol ether; sugars such as D-glucose; solubilizers, stabilizers, buffer substances, perfume oils and fragrances, dyes, and hair-conditioning and hair-care ingredients such as lanolin derivatives, cholesterol, pantothenic acid, and betaine.

### Cationic polymers

Useful are also a cationic copolymer, a cationic cellulose polymer or a cationic silicon polymer, where the quaternary nitrogen groups are contained either in the polymer chain or preferably as substituents on one or more of the monomers. The ammonium group-containing monomers can be copolymerized with non-cationic monomers. Suitable cationic monomers are unsaturated, radical polymerizable compounds carrying at least one cationic group, particularly ammonium-substituted vinyl monomers such as trialkyl methacryl oxyalkyl ammonium, trialkyl acryloxyalkyl ammonium, dialkyl diallyl ammonium, and quaternary vinyl ammonium monomers with groups containing cyclic, cationic nitrogen such as pyridinium, imidazolium, or quaternary, e.g. alkyl vinyl imidazolium, alkyl vinyl pyridinium, or alkyl vinyl pyrrolidone salts. The alkyl groups of these monomers are preferably lower alkyl groups, such as C1 to C7 alkyl groups, with C1 to C3 alkyl groups being especially preferred.

The ammonium group-containing monomers can be copolymerized with non-cationic monomers. Suitable co-monomers are, for example, acrylamide, methacrylamide, alkyl- and dialkyl acrylamide, alkyl- and dialkyl methacrylamide, alkyl acrylate, alkyl methacrylate, vinyl caprolactone, vinyl caprolactam, vinyl pyrrolidone, vinyl ester, e.g. vinyl acetate, vinyl alcohol, propylene glycol, or ethylene glycol, wherein the alkyl groups of these monomers are preferably C1 to C7 alkyl groups, with C1 to C3 alkyl groups being especially preferred.

Preferred polymers with quaternary amine groups are polymers selected from those described in the CTFA Cosmetic Ingredient Dictionary (10^{th} edition of 2004) under the designations as follows: the polymers sold by the company Rhodia Inc. under the trade names Mirapol^{®} A15 (POLYQUATERNIUM-2); Mirapol^{®} AD1 (POLYQUATERNIUM-17), Mirapol^{®} AZ1 (POLYQUATERNIUM-18) and Mirapol^{®} 175 (POLYQUATERNIUM-24); the copolymer of dimethyldiallylammonium chloride and acrylamide, sold by the company Ondeo Nalco under the trade names Merquat^{®} 550, Merquat^{®} 550L and Merquat^{®} S (POLYQUATERNIUM-7); quaternized hydroxyethyl cellulose (POLYQUATERNIUM-10); vinyl pyrrolidone/dimethylamino ethylmethacrylate methosulfate copolymer, sold under the trade names Gafquat^{®} 755 N and Gafquat^{®} 734 (POLYQUATERNIUM-11) by Gaf Corp. in the USA; Gafquat^{®} 734 is especially preferred; copolymer of acrylamide and betamethacrylyloxyethyl trimethyl ammonium chloride, sold by Rohm GmbH in Germany under the trade name Rohagit^{®} KF 720 (POLYQUATERNIUM-1 5); a copolymer sold by BASF in Germany under the trade name LUVIQUAT^{®} FC 550, LUVIQUAT^{®} FC905 and LUVIQUAT^{®} HM 552 consisting of polyvinyl pyrrolidone and imidazolimine methochloride (POLYQUATERNIUM-16); the copolymer of diallyldimethylammonium chloride and acrylic acid (80/20), sold by the company Calgon under the trade name Merquat^{®} 280 (POLYQUATERNIUM-22); polymers sold under the trade name Quatrisoft^{®} LM 200 by the company Amerchol, defined as polymeric quaternary ammonium salt of hydroxyethyl cellulose reacted with a lauryl dimethyl ammonium substituted epoxide (POLYQUATERNIUM-24); the copolymer of acrylamide and dimethylaminoethyl methacrylate (20/80 by weight) quaternized with methyl chloride, sold as a 50% by weight dispersion in a mineral oil under the trade name Salcare^{®} SC92 by the company Ciba Specialty Chemicals (POLYQUATERNIUM 32); the methosulfate of the copolymer of methacryloyloxyethyl trimethylammonium and methacryloyloxyethyl dimethylacetylammonium, sold under the trade name Plex^{®} 7525L and Plex^{®} 3074L by the company Rohm GmbH/Germany (POLYQUATERNIUM-35); a terpolymer sold by Calgon in the USA under the trade name Merquat^{®} Plus 3300 consisting of dimethyl diallyl ammonium chloride, sodium acrylate and acrylamide (POLYQUATERNIUM-39); a terpolymer from ISP in the USA, sold under the trade name Gaffix^{®} VC 713 consisting of vinyl pyrrolidone, dimethylamino ethyl methacrylate, and vinyl caprolactam; and the copolymer sold by the company Gaf under the trade name Gafquat^{®} HS 100 consisting of vinyl pyrrolidone/methacrylamidopropyltrimethyl ammonium chloride; the cationic polymer sold under the trade name Polymer SL^{®}-60 (CTFA: POLYQUATERNIUM-67) by the company Amerchol.

Suitable cationic polymers that are derived from natural polymers are cationic derivatives of polysaccharides, for example, cationic derivatives of cellulose, starch, or guar. Furthermore, chitosan and chitosan derivatives are suitable. Suitable cationic polysaccharides display the general formula

G-O-B-N⁺R^{a}R^{b}R^{c} Y (III)

G is an anhydroglucose residue, for example, starch or cellulose anhydroglucose; B is a divalent bonding group, for example alkylene, oxyalkylene, polyoxyalkylene or hydroxy-alkylene;
R^{a} R^{b}, and R^{c} are independently from one another alkyl, aryl, alkylaryl, arylalkyl, alkoxyalkyl, or alkoxyaryl each having 1 to 18 C atoms, wherein the total number of C atoms in R^{a}, R^{b}, and R^{c} is preferably a maximum of 20;
Y⁻ is an anion, preferably chloride, bromide, sulfate or methosulfate.

A suitable cationic cellulose is sold by Amerchol under the trade name Polymer^{®} JR and has the INCl and CTFA designation POLYQUATERNIUM-1 0. A further cationic cellulose has the INCl and CTFA designation POLYQUATERNIUM-24 and is sold by Amerchol under the trade name Polymer^{®} LM-200. A suitable cationic guar derivative is sold under the trade name Jaguar^{®} R and has the INCl and CTFA designation Guar Hydroxypropyltrimonium Chloride.

Other suitable cationic polymers are chitosan, chitosan salts, and chitosan derivatives. The chitosans are completely or partially de-acetylated chitins (POLYQUATERNIUM-29). To produce a chitosan, one preferably starts with the chitin contained in the shell residues of crustaceans, which, as a cheaper and natural raw material, is available in large quantities. The molecular weight of the chitosan can be distributed across a broad spectrum, for example, of from 20,000 to approx. 5 million g/mol (700 to about 176,000 oz/mol). For example, a suitable low-molecular-weight chitosan is one with a molecular weight of from 30,000 to 70,000 g/mol (1,100 to 25,000 oz/mol). Preferably, however, the molecular weight is greater than 100,000 g/mol (3,527 oz/mol), or especially preferred is a molecular weight of from 200,000 to 700,000 g/mol (7,054 to 24,692 oz/mol). The level of deacetylation is preferably from 10 to 99% by weight, with 60 to 99% by weight being especially preferred.

A suitable chitosan is sold, for example, by Kyowa Oil&Fat in Japan under the trade name Flonac^{®}. It has a molecular weight of 300,000 to 700,000 g/mol (10,582 to 24,692 oz/mol) and is deacetylated to 70 to 80%. A preferred chitosan salt is chitosonium pyrrolidone carboxylate, which, for example, is sold under the name Kytamer^{®} PC by Amerchol in the USA. The chitosan contained therein has a molecular weight of approx. 200,000 to 300,000 g/mol (7,054 to 10,582 oz/mol) and is deacetylated to 70 to 85%. Quaternized, alkylated, or hydroxyalkylated derivatives, for example, hydroxyethyl or hydroxybutyl chitosans can be considered as useful chitosan derivatives.

The chitosans or chitosan derivatives are preferably present in their neutralized or partially neutralized form. The level of neutralization for the chitosan or the chitosan derivative is preferably at least 50% by weight, with 70 to 100% by weight being especially preferred, based on the number of free base groups. In principle, all cosmetically compatible inorganic or organic acids can be used as neutralizers such as formic acid, malic acid, succinic acid, tartaric acid, citric acid, malonic acid, oxalic acid, and pyrrolidone carboxylic acid, of which the citric acid is preferred.

Other suitable cationic polymers that can be contained in the permanent shaping composition according to the present invention are cationic modified protein derivatives or cationic modified protein hydrolysates and are, for example, known under the INCl designations Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, or Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, and Hydroxypropyltrimonium Hydrolyzed Vegetable Protein.

Suitable cationically derived protein hydrolysates are substance mixtures, which, for example, receive glycidyl trialkyl ammonium salts or 3-halo-2-hydroxypropyl trialkyl ammonium salts via the conversion of alkaline, acidic, or enzyme-hydrolyzed proteins. Proteins that are used as starting materials for the protein hydrolysates can be of plant or animal origin. Customary starting materials are, for example, keratin, collagen, elastin, soy protein, rice protein, milk protein, wheat protein, silk protein, or almond protein. The hydrolysis results in material mixtures with mole masses in the range of approximately 100 to approx. 50,000. Customary, mean mole masses are in the range of about 500 to about 1,000. It is advantageous if the cationically derived protein hydrolysates have one or two long C8 to C22 alkyl chains and two or one short C1 to C4 alkyl chain accordingly. Compounds containing one long alkyl chain are preferred.

Suitable cationic silicon polymers either have at least one least one ammonium group, examples are POLYSILICONE-9; preferred are diquaternary polysiloxanes as defined in the EP 0714654 A1 of the applicant and those having the chemical name Dimethylsiloxane, 3-(3-((3-cocoamidopropyl)dimethylammonio)-2-hydroxy-prpoxy)propyl groupterminated acetate (CAS 134737-05-6), defined in the CTFA as QUATERNIUM-80 and sold under the trade names Abil Quat^{®} 3270, Abil^{®} Quat 3272 and Abil^{®} Quat 3474 by the company Th. Goldschmidt AG, Germany.

The most preferred cationic polymers which may be comprised in the composition of the invention are cationic polymers selected from the group consisting of the polymer of dimethyl diallyl ammonium chloride (CTFA: POLYQUATERNIUM-6); vinylpyrrolidon/dimethylaminoethylmethacrylat methosulfate (CTFA: POLYQUATERNIUM-11); aminoethylaminopropylsiloxane/dimethylsiloxan-copolymer emulsion; quaternized vinylpyrrolidon/dimethylaminoethylmethacrylate-copolymer; copolymer of methylvinylimidazoliumchloride and vinylpyrrolidone (CTFA: POLYQUATERNIUM-16); the quaternary ammonium salt of the terpolymer of acrylic acid/diallyldimethylammonium chloride/acrylamide; ethanaminium, N,N,N-trimethyl-2-[(methyl-1-oxo-2-propenyl)oxy]-chloride homopolymer (CTFA: POLYQUATERNIUM-37); the copolymer of hydroxyethyl cellulose and diallyldimethylammonium chloride; the copolymer of acrylamide and diallyldimethylammonium chloride (CTFA: POLYQUATERNIUM-7); the copolymer of acrylamide and betamethacrylyloxyethyl trimethyl ammonium chloride (CTFA: POLYQUATERNIUM-5); the copolymer of methacrylamide and betamethacrylyloxyethyl trimethyl ammonium chloride (CTFA: POLYQUATERNIUM-15); the copolymer of polyvinyl pyrrolidone and imidazolimine methochloride; the copolymer of diallyldimethyl ammonium chloride and acrylic acid (CTFA: POLYQUATERNIUM-22); the copolymer of vinyl pyrrolidone and methacrylamidopropyl trimethyl ammonium chloride (CTFA: POLYQUATERNIUM-28); the methosulfate of the copolymer of methacryloyloxyethyl trimethylammonium and methacryloyloxyethyl dimethylacetylammonium, quaternized hydroxyethyl cellulose (CTFA: POLYQUATERNIUM-10); copolymer of hydroxyethyl cellulose and diallyldimethylammonium chloride (CTFA: POLYQUATERNIUM-4); dimethylsiloxane 3-(3-((3-cocoamidopropyl)dimethylammonio)-2-hydroxyprpoxy)propyl groupterminated acetate; dimethylsiloxane, 3-(3-((3-cocoamidopropyl)dimethylammonio)-2-hydroxyprpoxy)propyl groupterminated acetate (CTFA: QUATERNIUM-80); N-(3-chloroallyl)hexaminium chloride (QUATERNIUM-15); the polyethylene glycol derivative of aminoethylaminopropylsiloxane/dimethylsiloxan-copolymer; a quaternary ammonium compound sold under the trade name Finquat^{®} CT by the company Finetex (CTFA: QUATERNIUM-75; see CTFA 10th Edition, page 1604) and cationic silicone polymers.

The composition of the invention comprises the cationic monomer in an amount of from 0.05% to 15% by weight, preferably from 0.1 % to 6% by weight, most preferred from 0.5% to 3% by weight of the composition polymers.

### Silicone Conditioning Agents

The permanent hair shaping compositions hereof can also include volatile or nonvolatile, soluble or insoluble silicones as conditioning agents. By soluble what is meant is that the silicone conditioning agent is miscible with the aqueous carrier of the composition so as to form part of the same phase. By insoluble what is meant is that the silicone forms a separate, discontinuous phase from the aqueous carrier, such as in the form of an emulsion or a suspension of droplets of the silicone.

Soluble silicones include silicone copolyols, such as dimethicone copolyols, e.g. polyether siloxane-modified polymers, such as polypropylene oxide, polyethylene oxide modified polydimethylsiloxane, wherein the level of ethylene and/or propylene oxide sufficient to allow solubility in the composition.

Preferred, however, are insoluble silicones. The insoluble silicone hair conditioning agent for use herein will preferably have viscosity of from about 1,000 to about 2,000,000 mPa·s at 25°C, more preferably from about 10,000 to about 1,800,000, even more preferably from about 100,000 to about 1,500,000 mPa · s at 25°C. The viscosity can be measured by means of a glass capillary viscometer as set forth in Dow Corning Corporate Test Method CTM0004, July 20, 1970.

Suitable insoluble, nonvolatile silicone fluids include polyalkyl siloxanes, polyaryl siloxanes, polyalkylaryl siloxanes, polyether siloxane copolymers, dimethylpolysiloxane containing terminal hydroxyl groups, methylphenyl polysiloxane containing terminal hydroxyl groups and mixtures thereof. Specific examples thereof include hexamethyldisiloxane, octamethyltrisiloxane, decamethyltetrasiloxane and hexadecamethylheptasiloxane.

Other insoluble, nonvolatile silicone fluids having hair conditioning properties can also be used. The term "nonvolatile" as used herein shall mean that the silicone has a boiling point of at least about 260°C, preferably at least about 275°C, more preferably at least about 300°C. Such materials exhibit very low or no significant vapor pressure at ambient conditions. The term "silicone fluid" shall mean silicone materials capable of flowing and having a viscosity of less than 1,000,000 mPa · s at 25°C. Generally, the viscosity of the fluid will be between about 5 and 1,000,000 mPa · s at 25°C, preferably between about 10 and about 300,000 mPa · s at 25°C.

The preferred silicones are polydimethyl siloxane, polydiethylsiloxane, and polymethylphenylsiloxane. Polydimethylsiloxane is especially preferred. The nonvolatile polyalkylsiloxane fluids that may be used include, for example, polydimethyl siloxanes. These siloxanes are available, for example, from the General Electric Company in their ViscasilR^{®} and SF^{®} 96 series, and from Dow Corning in their Dow Corning 200^{®} series.

The polyalkylaryl siloxane fluids that may be used, also include, for example, polymethylphenylsiloxanes. These siloxanes are available, for example, from the General Electric Company as SF 1075 methyl phenyl fluid or from Dow Corning as 556 Cosmetic Grade Fluid.

Suitable insoluble, volatile silicone fluids include low molecular weight oligomeric polydimethylsiloxane or cyclic polydimethylsiloxane, having a viscosity of no more than 10 mPa · s at 25°C and a boiling point under atmospheric pressure of no more than 250°C. Volatility can be achieved in linear organopolysiloxanes by selection of oligomeric organopolysiloxanes with at most 6 to 10 silicone atoms in the organopolysiloxanes backbone, e. g. Dow Corning DC200 Fluid^{®}, having a viscosity of from about 0.65 to about 2 mPa · s at 25°C. Preferably cyclic organopolysiloxanes having from 3 to 6 silicon atoms are utilized, for example, hexamethylcyclotrisiloxane, octamethylcyclotetrasiloxane, decamethylcyclopentasiloxane, dodecamethylcyclohexasiloxane (e. g. DC 244, DC 245, DC 345 of Dow Corning).

Especially preferred, for enhancing the shine characteristics of hair, are highly arylated silicones, such as highly phenylated polyethyl silicone having refractive indices of about 1.46 or higher, especially about 1.52 or higher. When these high refractive index silicones are used, they should be mixed with a spreading agent, such as a surfactant or a silicone resin, as described below to decrease the surface tension and enhance the film forming ability of the material.

The polyether siloxane copolymers that may be used include, for example, a polypropylene oxide modified polydimethylsiloxane (e. g. Dow Corning DC-1248^{®}) although ethylene oxide or mixtures of ethylene oxide and propylene oxide may also be used. The ethylene oxide and polypropylene oxide level should be sufficiently low to prevent solubility in the composition hereof.

Another silicone hair conditioning material that can be especially useful in the silicone conditioning agents is insoluble silicone gum. The term "silicone gum", as used herein, means polyorganosiloxane materials having a viscosity at 25°C of greater than or equal to 1,000,000 mPa · s. The "silicone gums" will typically have a mass molecular weight in excess of about 200,000, generally between about 200,000 and about 1,000,000. Specific examples include polydimethylsiloxane, (polydimethylsiloxane) (methylvinylsiloxane) copolymer, poly(dimethylsiloxane) (diphenyl siloxane) (methylvinylsiloxane) copolymer and mixtures thereof. Preferably the silicone hair treating agent comprises a mixture of a polydimethylsiloxane gum, having a viscosity greater than about 1,000,000 mPa · s and polydimethylsiloxane fluid having a viscosity of from about 10 mPa · s to about 100,000 mPa · s at 25°C, wherein the ratio of gum to fluid is from about 30:70 to about 70:30, preferably from about 40:60 to about 60:40.

Further silicones for use herein which are preferred are amino functional siloxanes which conform to the general formula (IV) 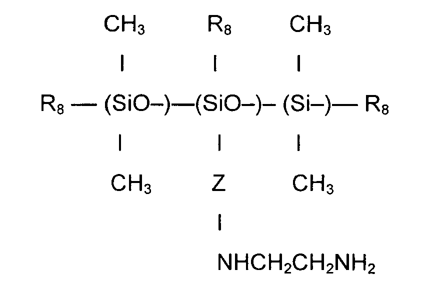 wherein R₈ = OH or CH₃ and Z represents the propyl, isopropyl or isobutyl group. These silicones, e. g., copolymer of amino ethyl amino propyl siloxane and dimethyl siloxane are available from Dow Corning and sold under the trade name Dow Corning 2-8566 Amino Fluid^{®} or as a mixture with polyethylenglycol ether of tridecyl alcohol and cetyl trimethyl ammonium chloride, sold as Dow Corning 929 Cationic Emulsion^{®}.

Silicone polymers that are specially preferred are CTFA: QUATERNIUM-80 and AMODIMETHICONE. Also preferred are volatile silicones such as e. g. CTFA: DIMETHICONE and CYCLOMETHICONE.

The silicone hair conditioning agent can be used in the compositions hereof at levels of from about 0.1 % by weight to about 20% by weight of the composition, preferably from about 0.5 % by weight to about 15% by weight, more preferably from about 1% by weight to about 10% by weight and most preferably from about 3% by weight to about 8% by weight by weight.

### Additional Conditioning Agents

The cosmetic hair shaping compositions of the present invention can also comprise one or more additional conditioning agents, such as those selected from the group consisting of liquid oils and fats such as avocado oil, tsubaki oil, turtle oil, macademia nuts oil, corn oil, mink oil, olive oil, rape seed oil, yolk oil, sesame oil, parsic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, hohoba oil, germ oil, triglycerine, trioctanoic acid glycerine, triisopalmitic acid glycerine; solid fats such as cacao fat, coconut oil, horse fat, hardened coconut fat, palm oil, tallow, sheep fat, hardened tallow, palm kernel oil, jojoba oil, lard, ox bone fat, wood wax kernel oil, hardened castor oil; waxes such as beeswax, apple wax, canderilla wax, cotton wax, carunauba wax, bayberry wax, insect wax, whale wax, montan wax, rice bran wax, lanolin, kapok wax, lanolin acetate, liquid lanolin, cane wax, isopropyl lanolin fatty acid, hexyllaurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether; hydrocarbons , nonvolatile hydrocarbons and hydrocarbon esters such as fluid paraffin, solid paraffin, vaseline, ozocerite, squalane, pristan, ceresin, squalane, petrolatum, isododecane, microcrystalline wax; fatty acid oils, ester oils such as cetyl octanoate, isopropyl myristate; betaine, carnitin, carnitin esters, creatine, amino acids, peptides, proteins, vitamins, phospholipids, e. g. lecithines or ceramides. Useful are also imidazolidinyl derivatives as for example (CTFA: QUATERNIUM-87) sold under the trade name Rewoquat^{®} W 575 by Witco, Germany.

The amount of fatty substances preferably ranges from about 0.5 to about 30% by weight, better still from about 1 to about 15% by weight and most preferred from about 2 to about 10% by weight of the total weight of the composition. These ranges include all specific values and sub-ranges there between, including 3, 8, 10, 15, 20, 25 and 30% by weight.

### Fatty Alcohols

The cosmetic compositions of the present invention may also comprise at least one nonvolatile low melting point fatty alcohol. The fatty alcohols hereof have a melting point of 30°C or less, preferably about 25°C or less, more preferably about 22°C or less. The unsaturated fatty alcohols hereof are also nonvolatile. By nonvolatile what is meant is they have a boiling point at 1.0 atmospheres of at least about 260°C, preferably at least about 275°C, more preferably at least about 300°C. Suitable fatty alcohols include unsaturated monohydric straight chain fatty alcohols, saturated branched chain fatty alcohols, saturated C8-C12 straight chain fatty alcohols, and mixtures thereof. The unsaturated straight chain fatty alcohols will typically have one degree of unsaturation. Di- and tri- unsaturated alkenyl chains may be present at low levels, preferably less than about 5% by total weight of the unsaturated straight chain fatty alcohol more preferably less than about 2% by weight, most preferably less than about 1% by weight. Preferably, the unsaturated straight chain fatty alcohols will have an aliphatic chain size of from C12-C22, more preferably from C12-C18, most preferably from C16-C18. Exemplary alcohols of this type include oleyl alcohol, and palmitoleic alcohol. The branched chain alcohols will typically have aliphatic chain sizes of from C12-C22, preferably C14-C20, more preferably C16-C18.

Exemplary branched chain alcohols for use herein include stearyl alcohol, cetyl alcohol, isostearyl alcohol, octyl dodecanol, and octyl decanol. Examples of saturated C8-C12 straight chain alcohols include octyl alcohol, caprylic alcohol, decyl alcohol, and lauryl alcohol. The low melting point fatty alcohols hereof are used at a level of from about 0.1 % by weight to about 10% by weight of the composition, more preferably from about 0.2% by weight to about 8% by weight, most preferably from about 0.5% by weight to about 6% by weight.

It may be desirable to use waxy fatty alcohols for their conditioning benefits. In the event that such saturated fatty alcohols are present, the weight ratio of the liquid to waxy fatty alcohols is preferably no greater than about 0.25, more preferably no greater than about 0.15, more preferably than about 0.10. The total amount of fatty alcohols in the composition is preferably about 0.5 to about 10% by weight, more preferably from about 1.0 to about 7% by weight, and most preferably from about 2 to about 5% by weight.

### Other Ingredients

The cosmetic compositions herein can contain a variety of other optional components suitable for rendering such compositions more cosmetically or aesthetically acceptable or to provide them with additional usage benefits. Such conventional optional ingredients are well-known to those skilled in the art. The composition of the invention may thus comprise lipophilic or hydrophilic adjuvants which are standard in the cosmetics or dermatological fields, e.g. screening agents, odor absorbers, coloring materials and lipid vesicles.

A wide variety of additional ingredients can be formulated into the present cosmetic composition. These include: hair-hold polymers, additional thickening agents and suspending agents, viscosity modifiers such as methanolamides of long chain fatty acids, e.g. cocomonoethanol amide, crystalline suspending agents; pearlescent aids such as ethylene glycol distearate; UV-filters and sunscreens, e. g. such as p-methoxy cinnamic acid isoamylester, lipophilc cinnamic acid esters, salicylic acid esters, 4-amino benzoic acid derivatives or hydrophilic sulfonic acid derivatives of benzophenones or 3-benzyliden campher; antioxidants such as tocopheroles; agents for combating free radicals; preservatives such as benzyl alcohol, methyl paraben, propyl paraben and imidazolidinyl urea; polyvinyl alcohol; acidic pH adjusting agents, such as citric acid, formic acid, glyoxylic acid, acetic acid, lactic acid, pyruvic acid, sodium citrate, succinic acid, phosphoric acid; salts, in general, such as sodium carbonate; sodium and potassium acetate, sodium and potassium chloride or sulfate; coloring agents, such as any of the FD&C or D&C dyes; perfumes, sequestering agents, such as disodium ethylenediamine tetra-acetate, and polymer plasticizing agents, such as glycerin, disobutyl adipate, and butyl stearate.

From the nonionic surfactants optionally used those are preferred having an HLB (Hydrophilic Lipophilic Balance) of greater than 12 and the primary emulsion comprises at least one nonionic surfactant having an HLB of less than 8. The nonionic surfactant of HLB>12 optionally present in the emulsion may be, for example, an ethoxylated or ethoxylated/propoxylated fatty alcohol with a fatty chain comprising from 12 to 22 carbon atoms, ethoxylated sterols, such as stearyl- or lauryl alcohol (EO-7); PEG-16 soya sterol or PEG-10 soya sterol, polyoxyethylene polyoxypropylene block polymers (poloxamers) and their mixtures. Ethoxylated sterols and of poloxamers are preferred. The nonionic surfactant of HLB<8 can be chosen in particular from glyceryl esters, such as mono-, di- or triglyceryl mono-, di- or tri-isostearate or -oleate, sugar esters, such as sucrose or methyl glucose mono- or diisostearate or -oleate, alkylpolyglucoside ethers, such as sorbitan isostearate, oleyl- or isostearylpolyglucoside; polyoxyethylene (20) sorbitan monostearate (CTFA: POLYSORBATE-60), and their mixtures. Sugar esters and alkylpolyglucoside ethers are preferred.

The amount of nonionic surfactant in the cosmetic composition preferably can range from about 0.1 to about 5% by weight and more preferably from about 1 to about 3% by weight of the total weight of the emulsion.

The additives are used in quantities usual for such purposes; for example, the wetting agents and emulsifiers in concentrations of a total of 0.2 to 30 % by weight, the alcohols in a total quantity of 0.1 to 20 % by weight, the opacifiers, perfume oils and dyes in a quantity of 0.01 to 1 % by weight each, the buffer substances in a total quantity of 0.1 to 10 % by weight, and sugars, solubilizers, stabilizers and hair-conditioning and hair-care ingredients in a quantity of 0.1 to 5 % by weight each, while the thickeners and solubilizers may be contained in this composition in a total quantity of 0.5 to 20 % by weight. Non-cationic surfactants are preferably contained at levels of from about 0.1 % by weight to about 5% by weight, more preferably from about 0.2% by weight to about 1,5% by weight, most preferably from about 0.4% by weight to about 0.8% by weight, by weight of the composition.

### pH-Value

By varying the pH value, a composition can be made available that is universally suitable for every hair structure, optionally with the additional application of heat. The composition brings about an elastic, durable and uniform waving from the root of the hair to the ends, without eliciting allergic or sensitizing reactions.

The hair shaping composition preferably has a pH value of from 2.0 to 9.5, more preferably from 4.0 to 9.0 and most preferred from 5.5 to 8.5. To establish an acidic pH value, hydrochloric acid, phosphoric acid, acetic acid, lactic acid or citric acid can be used in particular. The acidic pH can be also adjusted with a buffer such as a phosphate buffer, a TRIS buffer or a citric buffer. The buffers may be used alone or in combination with an acid.

If the shaping composition is adjusted to be acidic (for example to a pH = 5.0 to 6.9), then esters of mercapto carboxylic acids may be present as co-agents such as, for example, monothioglycol acid glycol esters or -glycerin esters, with mercapto acetamides or 2-mercaptopropionic acid amides being preferred, in a concentration of from 1 to 8 % by weight; or the salts of the sulfuric acid, for example, sodium, ammonium, or monoethanol ammonium sulfite, in a concentration of from 3 to 8 percent by weight (calculated as SO₂).

### Viscosity

The hair shaping composition of the present invention preferably has a viscosity at 25°C of about 0.1 mPa·s to about 10,000 mPa·s, preferably from about 1 mPa·s to about 3,000 mPa·s, more preferably from about 2 mPa·s to about 1,000 mPa·s. Viscosity is determined - if not otherwise defined - by a HAAKE Rotation Viscometer VT 550 with cooling/heating vessel and sensor systems according to DIN 53019 (NV-DIN, SV-DIN), the shear rate is 12.9 s⁻¹.

### Packages

The shaping composition may be sold in single- or dual- or triple-component packages; the composition may be in the form of an aqueous solution, or an emulsion, or an aerosol foam, or in thickened water-based form, especially a creme, gel, foam or paste.

### Method for Permanent Hair Shaping

The method of permanent shaping of human hair according to the invention is defined in claim 20.

Further details of the method are given as follows: First the hair (which is washed and towel-dried) is separated into multiple sections and then these sections are rolled onto curlers. The curlers used for permanent waves have a diameter of about 5 to 13 mm (0.17 to 0.44 in), while the curlers used for straightening must have a diameter greater than 13 mm (0.44 in). After the rolling on curlers is finished, the curlers are thoroughly wetted down using the required quantity of the permanent shaping composition, preferably 60 to 120 g.

The amount of time the permanent shaping composition stays on the hair is from about 1 to about 30 minutes, preferably from about 2 to about 20 minutes, most preferred 8 to 15 minutes. This action time can be shortened by adding heat via the use of a heat radiator or a hood dryer.

After the action time has elapsed that is sufficient for the permanent shaping, which is depending on hair quality, the pH value, the shaping effectiveness of the shaping composition, the desired level of change as well as on the application temperature, the hair is rinsed with water or treated with an intermediate treatment composition. Thereafter the hair is oxidatively post-treated ("fixed"). The fixing composition is used in a quantity of about 50 to 200 g (1.76 to 7.05 oz), preferably 80 to 100 g (2.27 to 3.52 oz), depending on hair thickness and length. Any oxidizing composition that has been used before in fixing compositions can be used for the fixation. Examples of such oxidizing agents are potassium bromate, sodium bromate, sodium perborate, dehydroascorbic acid, hydrogen peroxide, and urea peroxide. Hydrogen peroxide is preferred. The concentration of the oxidizing agent varies depending on application time (normally 3 to 15 minutes, or preferably 5 to 10 minutes) and application temperature (25 to 50°C (77 °F to 122 °F)).

The concentration of the oxidant varies, depending on the application time (as a rule, 5 to 10 minutes) and the application temperature. Normally, oxidizing agents are used in a concentration of from about 0.5 to about 12.0 % by weight, preferably from about 1 to about 3 % by weight, in the aqueous fixing composition.

The fixing composition can obviously contain other materials, for example, weak acids or peroxide stabilizers. The fixing composition can naturally also include other substances, such as wetting agents, hair-care substances such as cationic polymers, weak acids, buffer substances or peroxide stabilizers. These typical additives may be contained in the fixing composition in a quantity of from about 0.1 to about 10 % by weight, in particular.

Both the permanent shaping composition of the invention and the fixing composition can be present in the form of an aqueous solution or an emulsion, as well as in a thickened form on an aqueous basis, particularly as a cream, gel, or paste. It is also possible to fill these compositions into aerosol cans under pressure and to release them as aerosol foam. It is especially preferred that the fixing composition be in low viscosity liquid form. It is preferred that the fixing composition be an oxidation agent-containing, liquid preparation with a viscosity of from 1 to 500 mPa·s at 25°C (77 °F), wherein viscosity of from 1 to 10 mPa·s at 25°C (77 °F) is especially preferred. These viscosity values are based on measurements with a Haake rotational viscometer, type VT 501, at a shear speed of 64.5 per second.

After an action period required for the fixing composition of from about 3 to about 15 minutes, preferably 5 to 10 minutes, the curlers are removed. If necessary, the hair, after being taken down from the curlers, is oxidatively post-treated yet again, than the fixing composition is rinsed from the hair with water. The hair is then optionally treated with a known acidic rinse. It is advantageous if the hair is then finally shaped into a water wave and then dried.

It is completely surprising that the method and composition for permanent hair shaping of the invention leads to improved results in a number of respects compared to the compositions comrising solely the widely used shaping agent thioglycolic acid or its salt with ammonia, but mainly in that the smell of the hair treated is significantly better and the hair suffers less damage. Surprisingly also the odor during application of the compositions of the invention is significantly better compared to compositions on the basis of the widely used hair shaping agent thioglycolic acid or its salt with ammonia.

Surprisingly it was also observed that the uniformity of the hair shape wasn't reduced when the hair was exposed to humidity as e.g. sweat from the skin. The less resistance of the uniformity of the hair shape was stated when common compositions basing on thioglycolic acid and its salts, cysteamine, thiolactic acid and other reducing agents without N-alkyl-2-mercaptoacetamide were used.

The composition and method of the invention further does not have the disadvantages of glyceryl thioglycolate and thioglycolic acid and its salts but possesses the advantages of displaying no sensitization problem and no skin irritation, whereby showing superior hair care effects. The excellent condition of hair treated with composition and method of the invention was shown by an improved soft feel of wet and dry hair, an improved uniformity of the curls from the roots to the tips, an improved elasticity of hair and durable waving of the hair.

The following examples are intended to explain the subject of the invention in further detail, but without limiting the subject to these examples.

### Examples

### Example 1 Low Alkaline Permanent Waving Composition for Normal Hair

| | |
|---|---|
| 12.50 g | N-(3'-hydroxypropyl)-2-mercaptoacetamide (90 % by weight aqueous solution) |
| 4.00 g | tris(hydroxymethyl)aminomethane (CTFA: TROMETHAMINE) |
| 0.50 g | polydimethyldiallyammonium chloride |
| 0.50 g | cetyltrimethylammonium chloride (CTFA: CETRIMONIUM CHLORIDE) |
| 1.00 g | 1,3-butandiol |
| 1.50 g | 1,2-propylenglycol |
| 2.00 g | dipropylenglycol monoethylether |
| 3.50 g | urea |
| 1.00 g | castor oil, ethoxylated with 35 moles ethylene oxide Chremophor^{®} EL (CTFA: PEG-35 CASTOR OIL) |
| 1.00 g | coconat alcohol, ethoxylated with 10 moles ethylene oxide Genapol^{®} C 100 (CTFA: CocETH-10) |
| ad 100.00 g | water |

The pH of the composition is adjusted with tris(hydroxymethyl)aminomethane to 8.4

After winding the hair on curlers the above permanent waving composition is uniformly applied onto the curlers. The permanent waving composition is left on the curlers for 10 minutes. An infrared drying hood is used at a temperature of 40°C. During the treating time a much better and more pleasant smell is notices by the hairdresser as well as by the customer compared to permanent waving compositions commonly used. The hair is than rinsed with lukewarm water. Thereafter 80g of a fixing composition of the formula given below is applied onto the curler.

**Fixing Composition**

| | |
|---|---|
| 4.00 g | hydrogen peroxide, 50% by weight aqueous solution |
| 0.10 g | salicylic acid |
| 0.20 g | dinatriumhydrogenphosphate |
| 0.15 g | o-phosphoric acid |
| 1.00 g | castor oil ethoxylated wit 35 moles of ethylene oxide |
| 0.10 g | vinylpyrrolidon/styrene-copolymer |
| 0.10 g | perfume oil |
| ad 100.00 g | water |

The fixing composition is left on the hair for 6 minutes. Finally the hair is again rinsed with lukewarm water and the curlers are removed.

The thus-treated hair has a uniform, lively curliness and displays a good elasticity from the tips to the roots. It displays a good fastness, even over a long period after the treatment, to humidity particularly sweat. Even after 10 days the hair keeps its pleasant smell either in dry or in wet condition. Not the slightest skin irritation was noticed.

### Example 2: Low Alkaline Permanent Waving Composition for Colored Hair

| | |
|---|---|
| 4.00 g | N-(3'-hydroxypropyl)-2-mercaptoacetamide |
| 3.00 g | cystein hydrochloride |
| 0.20 g | ammonia (25% aqueous solution) |
| 3.50 g | ammonium hydrogen carbonate |
| 0.20 g | cetyltrimethyl ammonium bromide (CTFA: CETRIMONIUM BROMIDE) |
| 0.90 g | polydimethyldially ammonium chloride |
| 0.30 g | styrene/vinylpyrrolidon-copolymer (CTFA: STYRENE/VP COPOLYMER) |
| 1.50 g | 1,2-propylenglycol |
| 2.00 g | dipropyleneglycol monoethylether |
| 2.00 g | urea |
| 1.00 g | hydrogenated castor oil, ethoxylated wit 40 moles of ethylene oxide (CTFA: PEG-40 HYDROGENATED CASTOR OIL) |
| 1.00 g | Genapol^{®} C 100 (CTFA: COETH-10) |
| 0.50 g | perfume oil |
| ad 100.00 g | water |

The pH of the composition is adjusted with ammonia (25 % aqueous solution) to 8.2

After winding the hair on curlers the above permanent waving composition is uniformly applied onto the curlers. The permanent waving composition is left on the curlers for 10 minutes. An infrared drying hood is used at a temperature of 40°C. During the treating time a much better and more pleasant smell is notices by the hairdresser as well as by the customer compared to permanent waving compositions used commonly. The hair is than rinsed with lukewarm water. Thereafter 80g of a fixing composition of example 1 are applied onto the curler.

The fixing composition is left on the hair for 6 minutes. Finally the hair is again rinsed with lukewarm water and the curlers are removed. The hair exhibits a good elasticity and a uniform, lively curliness from the tips to the roots. It displays a good fastness, even over a long period after the treatment, to humidity particularly sweat. Even after 5 days the hair keeps its pleasant smell either in dry or in wet condition. Not the slightest skin irritations were noticed.

### Example 3 Low Alkaline Permanent Waving Composition for Colored Hair

| | |
|---|---|
| 5.50 g | N-(3'-hydroxypropyl)-2-mercaptoacetamide |
| 0.50 g | 2,2'-dithio-bis[(N-(3-hydroxypropyl)-acetamide] |
| 1.50 g | monoethanolamine |
| 0.50 g | CTFA: BEHENTRIMONIUM CHLORIDE |
| 1.00 g | aminofunctional siliconpolymer (CTFA: METHOXY PEG/PPG-7/3 AMINOPROPYL DIMETHICONE) |
| 0.30 g | styrene/vinylpyrrolidon-copolymer (CTFA: STYRENE/VP COPOLYMER) |
| 1.50 g | 1,2-butylenglycol |
| 2.50 g | dipropyleneglycol monoisopropylether |
| 2.00 g | urea |
| 1.00 g | hydrogenated castor oil, ethoxylated with 40 moles of ethylene oxide (CTFA: PEG-40 HYDROGENATED CASTOR OIL) |
| 1.00 g | Genapol^{®} C 100 (CTFA: COCETH-10) |
| 0.50 g | perfume oil |
| ad 100.00 g | water |

The pH of the composition is adjusted with monoethanolamine to 8.2.

After winding the hair on curlers the above permanent waving agent is uniformly applied onto the curlers. The permanent waving agent is left on the curlers for 12 minutes. An infrared drying hood is used at a temperature of 40°C. During the treating time a much better and more pleasant smell is noticed by the hairdresser as well as by the customer compared to permanent waving agents commonly used. The hair is than rinsed with lukewarm water. Thereafter 80g of a fixing agent of example 1 is applied onto the curler.

The fixing agent is left on the hair for 6 minutes. Finally the hair is again rinsed with lukewarm water and the curlers are removed. The hair shows an improved soft feel of wet and dry hair, an improved uniformity of the curls and an improved elasticity of hair from the roots to the tips. Even after 5 days the hair keeps its pleasant smell either in dry or in wet condition. It displays a good fastness, even over a long period after the treatment, to humidity particularly sweat. During or after the hair treating procedure described above neither irritations of the skin nor other effects on the skin were realized.

### Example 4 Low Alkaline Permanent Waving for Unmanageable Hair

| | |
|---|---|
| 14.00 g | N-(3'-hydroxypropyl)-2-mercaptoacetamide (90% by weight aqueous solution) |
| 5.00 g | monoethanolamine |
| 3.00 g | stearyltrimethylammonium chloride (CTFA: STEARTRIMONIUM CHLORIDE) |
| 0.30 g | quaternized vinylpyrrolidon/dimethylaminoethylmethacrylate-copolymer (POLYQUATERNIUM-11) |
| 1.00 g | 1,3-butanediol |
| 2.50 g | 1,2-propylenglycol |
| 2.00 g | dipropylenglycol monoethylether |
| 3.50 g | urea |
| 1.50 g | oleic alcohol, ethoxylated with 10 mol of ethylenoxide (CTFA: OLETH-10) |
| 1.00 g | cocos oil ethoxylated with 10 moles ethylenoxide Genapol^{®} C 100 (CTFA: COCETH-10) |
| 0.60 g | perfume oil |
| ad 100.00 g | water |

The pH of the composition is adjusted with monoethanolamine to 8.9

After winding the hair on curlers the above permanent waving composition is uniformly applied onto the curlers. An infrared drying hood is used at a temperature of 40°C. The permanent waving composition is left on the curlers for 14 minutes. During the treating time a much better and more pleasant smell is noticed by the hairdresser as well as by the customer compared to permanent waving compositions commonly used. The hair is than rinsed with lukewarm water. Thereafter 80g of a fixing composition of example 1 is applied onto the curler. The hair exhibits a good elasticity and a uniform lively curliness from the tips to the roots. It displays a good fastness to humidity particularly sweat even over a long period after the treatment. Even after 5 days the hair keeps its pleasant smell either in dry or in wet condition. Not the slightest skin irritations were noticed.

### Example 5 Acid 2-Component Drop Free Permanent Waving Composition for-Colored Hair

**Component 1**

| | |
|---|---|
| 0.10 g | Amerchol Polymer SL^{®}-60 (CTFA: POLYQUATERNIUM-67) |
| 0.60 g | octadecyl dimethyl benzyl ammoniumchloride (CTFA: STEARALKONIUM CHLORIDE) |
| 1.50 g | 1,2-propylenglycol |
| 3.00 g | urea |
| 1.80 g | oleic alcohol, ethoxylated with 20 mol of ethylenoxide (CTFA: OLETH-20) |
| 0.50 g | perfume oil |
| ad 100.00 g | water |

The pH of the component 1 is pre-adjusted with ammonia and hydrochloric acid to be 8.9 after mixing the two components.

**Component 2**

| | |
|---|---|
| 100.00 g | N-(3'-hydroxypropyl)-2-mercaptoacetamide (90% by weight aqueous solution) |

Immediately before use 60 g of Component 1 is mixed with 15g of Component 2.

The pH-value of the ready to use hair shaping composition is 6.8. 75 g of the ready-to-use hair shaping composition are uniformly applied on the hair wound on curlers of 8 mm diameter. The permanent waving composition is left on the curlers for 15 minutes at room temperature (25°C). During the treating time a much better and more pleasant smell is noticed by the hairdresser as well as by the customer compared to permanent waving compositions commonly used. The hair is than rinsed with lukewarm water. Thereafter 80g of a fixing composition of example 1 is applied onto the curler. The fixing composition is left on the hair for 5 minutes. Thereafter the hair is again rinsed with lukewarm water and the curlers are removed. The hair shows a well groomed look, a good touch, fine wet combing properties and a uniform curl all over the head. Even after 10 days the hair keeps its pleasant smell either in dry or in wet condition. It displays a good fastness to humidity particularly sweat, even over a long period after the treatment.

### Example 6 Permanent Waving Composition for Curling the Hair Re-Growth

| | |
|---|---|
| 12.50 g | N-(3'-hydroxypropyl)-2-mercaptoacetamide |
| 0.80 g | 2,2'-Dithiobis[N-(3-hydroxypropyl)-acetamide] |
| 1.50 g | monoethanolamine |
| 0.50 g | CTFA: BEHENTRIMONIUM CHLORIDE |
| 4.00 g | cetylstearyl alcohol (Lanette^{®} O) |
| 1.00 g | cetylstearyl alcohol polyethylenglycol (25) ether (Cremophor^{®} A 25) |
| 4.50 g | Glycerin |
| 0.10 g | Amerchol Polymer SL^{®}-60 (CTFA: POLYQUATERNIUM-67) |
| 0.50 g | perfume oil |
| ad 100.00 g | water |

The pH of the gel composition is adjusted with monoethanolamine to 8.6.

After winding the hair on curlers, the above permanent waving composition in form of a gel is uniformly applied with a brush on the re-growth (upper layer on curlers). The permanent waving composition is left on the curlers for 10 minutes at 25°C. During the treating time a much better and more pleasant smell is noticed by the hairdresser as well as by the customer compared to permanent waving compositions used commonly. The hair is than rinsed with lukewarm water. Thereafter 60g of a fixing composition of example 1 is applied onto the curler. The fixing composition is left on the hair for 10 minutes. Finally the hair is again rinsed with lukewarm water and the curlers are removed. The hair shows a good wave in the re-growth area, a good touch and excellent elasticity. Even after 10 days the hair keeps its pleasant smell either in dry or in wet condition. Not the slightest skin irritations were noticed.

### Example 7 2-Phase-Permanent Waving Composition for Equalizing Large Structural Differences on the Hair

**Phase 1**

| | |
|---|---|
| 5.40 g | N-(3'-hydroxypropyl)-2-mercaptoacetamide |
| 0.50 g | ammonia (25 % aqueous solution) |
| 2.00 g | ammonium hydrogen carbonate |
| 1.50 g | aminoethylaminopropylsiloxane/dimethylsiloxan-copolymer emulsion; Dow Corning 2-8566 ^{®} (CTFA: AMODIMETHICONE) |
| 0.10 g | behenyl dimethyl benzyl ammonium chloride |
| 5.00 g | 1,2-propylene glycol |
| 1.00 g | 1,3-butanediol |
| 2.00 g | dipropyleneglycol mono ethyl ether |
| 4.00 g | urea |
| 2.00 g | dipropylenglycol monoethylether |
| 1.00 g | hydrogenated castor oil, ethoxylated wit 35 moles of ethylene oxide (CTFA: PEG-35 HYDROGENATED CASTOR OIL) |
| 1.00 g | cocos oil ethoxylated with 10 moles ethylenoxide Genapol^{®} C 100 (CTFA: COCETH-10) |
| 0.30 g | creatinine |
| 0.30 g | styrene/vinyl pyrrolidone copolymer Antara^{®} 430 (CTFA: Styrene/VP Copolymer) |
| 0.50 g | perfume oil |
| ad 100.00 g | water |

The pH of the composition is adjusted with ammonia (25 % aqueous solution) to 8.2.

**Phase 2**

| | |
|---|---|
| 14.60 g | N-(3'-hydroxypropyl)-2-mercaptoacetamide |
| 0.50 g | ammonia (25 % aqueous solution) |
| 5.00 g | ammonium hydrogen carbonate |
| 4.00 g | cetylstearyl alcohol (Lanette^{®} O) |
| 0.50 g | behenyl dimethyl benzyl ammonium chloride (CTFA: BEHENALKONIUM CHLORIDE) |
| 1.50 g | Dimethylsiloxane, 3-(3-((3-cocoamidopropyl)dimethylammonio)-2-hydroxyprpoxy)propyl groupterminated acetate; Abil Quat^{®} 3474 (CTFA: QUATERNIUM-80; CAS 134737-05-6) |
| 0.50 g | perfume oil |
| ad 100.00 g | water |

The pH of the composition is 8.9

After winding the hair on curlers the above permanent waving liquid (Phase 1) of pH = 8.2, having a viscosity of 8 mPa · sec at 25°C, is uniformly applied onto the curlers. Immediately thereafter on each of the curlers the above permanent waving gel (Phase 2) of pH = 8.6, having a viscosity of 2,800 mPa · sec at 25°, is applied with a brush on the hair re-growth. The permanent waving composition is left on the curlers for 10 minutes at room temperature (25°C). During the treating time a much better and more pleasant smell is notices by the hairdresser as well as by the customer compared to permanent waving compositions commonly used. Than he hair is rinsed with lukewarm water. Thereafter 60g of a fixing composition of example 1 is applied onto the curler. The fixing composition is left on the hair for 8 minutes. Finally the hair is again rinsed with lukewarm water and the curlers are removed. The hair shows a well groomed look, a good touch, fine wet combing properties and a uniform curl all over the head. Even after 10 days the hair keeps its pleasant smell either in dry or in wet condition. It displays a good fastness, even over a long period after the treatment, to humidity particularly sweat. During or after the hair treating procedure described above neither irritations of the skin nor other effects on the skin were realized.

### Example 8 Aerosol Permanent Waving Cream for Curling the Hair Re-growth

| | |
|---|---|
| 13.00 g | N-(3'-hydroxypropyl)-2-mercaptoacetamide (90 % by weight aqueous solution) |
| 0.90 g | ammonia (25 % aqueous solution) |
| 5.00 g | ammonium hydrogen carbonate |
| 4.00 g | cetylstearyl alcohol (Lanette^{®} O) |
| 1.00 g | cetylstearyl alcohol polyethylenglycol (25) ether (Cremophor^{®} A 25) |
| 2.50 g | cetyldimethylhydroxyethyl ammoniumphosphate (CTFA: HYDROXYETHYL CETYLDIMONIUM PHOSPHATE) |
| 0.10 g | behenyl trimethyl ammonium chloride (Genamin^{®} KDMP) |
| 0.50 g | Dimethylsiloxane, 3-(3-((3-cocoamidopropyl)dimethylammonio)-2-hydroxyprpoxy)propyl group terminated acetate; (Abil Quat^{®} 3474) (CTFA: QUATERNIUM-80; CAS 134737-05-6) |
| 0.50 g | perfume oil |
| ad 100.00 g | water |

### Compoundinq:

Although all usual propellants may be used, dimethyl ether is preferred. The above creme is blended with the same weight amount (50 : 50) of the propellant dimethyl ether in a conventional container of 150 ml volume. A valve with a valve diameter of 0.5 mm and a valve cone of 0.25 mm is used to close the container.

### Application Procedure

After winding the hair on curlers, the above permanent waving composition in form of a creme is uniformly applied from the aerosol container through the spray nozzle onto on the hair re-growth (upper layer of the curlers). The permanent waving cream is left on the curlers for 10 minutes at 25°C. During the treating time a much better and more pleasant smell is notices by the hairdresser as well as by the customer compared to permanent waving compositions commonly used. The hair is than rinsed with lukewarm water. Thereafter 80g of a fixing composition of example 1 is applied onto the curlers. The fixing composition is left on the hair for 8 minutes. Than the hair is again rinsed with lukewarm water and the curlers are removed.

Since only the hair re-growth was curled the hair now exhibits a uniform curl from the tips to the roots and has an excellent elasticity. The hair tips show a good structural condition.

The hair shows a good wave in the re-growth area, a good touch and excellent elasticity. Even after 10 days the hair keeps its pleasant smell either in dry or in wet condition. Not the slightest skin irritations were noticed. Further the customer benefits from the drop fastness of the waving cream.

### Example 9 Permanent Waving Composition for Normal Hair

| | |
|---|---|
| 7.50 g | N-propyl-2-mercaptoacetamide |
| 4.00 g | Thioglycolic acid |
| 2.00 g | tris-(2-hydroxypropyl)amine |
| 0.20 g | polydimethyldially ammonium chloride |
| 1.50 g | stearyl amidopropyl dimethylamine |
| 5.00 g | 1,2-propylene glycol |
| 1.00 g | 1,3-butanediol |
| 2.00 g | dipropyleneglycol monoethyl ether |
| 4.00 g | urea |
| 2.50 g | hydrogenated castor oil, ethoxylated with 40 mole ethylene oxide |
| 2.50 g | lauryl alcohol, ethoxylated with 40 mole ethylene oxide (CTFA: Laureth-4) |
| 0.10 g | vinylpyrrolidone/styrene copolymer (Antara.sup.R 430, GAF Corp., New York) |
| 0.50 g | perfume oil |
| ad 100.00 g | Water |

The pH of this composition is adjusted with tris-(2-hydroxypropyl)amine to 8.6.

Normal hair, not previously damaged, is washed, dried with a hand towel, and wound onto curlers with a diameter of 6 mm. After that the hair is moistened thoroughly and evenly with the above-described hair waving composition. After an action time of 15 minutes, the hair is rinsed thoroughly with water and then oxidatively post-treated with 80 g of a 3% by weight aqueous hydrogen peroxide solution. After the curlers are taken out, the hair is rinsed again with water, set for a water wave, and then dried. The thus-treated hair has a uniform, lively curliness. It displays a good fastness to humidity, particularly sweat, even over a long period after the treatment. Even after 10 days the hair keeps its pleasant smell either in dry or in wet condition. Not the slightest skin irritation was noticed.

### Example 10 Low Alkaline Permanent Waving Composition for Normal Hair

| | |
|---|---|
| 11.00 g | N-(3'-hydroxypropyl)-2-mercaptoacetamide (90 % by weight aqueous solution) |
| 2.00 g | ammonium thioglycolate |
| 0.90 g | ammonia (25 % aqueous solution) |
| 5.00 g | ammonium hydrogen carbonate |
| 1.90 g | CTFA: DITALLOWETHYL HYDROXYETHYLMONIUM METHOSULFATE |
| 0.10 g | Amerchol Polymer SL^{®}-60 (CTFA: POLYQUATERNIUM-67) |
| 1.00 g | 1,3-butandiol |
| 1.50 g | 1,2-propylenglycol |
| 2.00 g | dipropylenglycol monoethylether |
| 3.50 g | urea |
| 1.00 g | coconat alcohol, ethoxylated with 10 moles ethylene oxide Genapol^{®} C 100 (CTFA: COCETH-10) |
| ad 100.00 g | water |

The pH of this composition is adjusted with ammonia (25 % aqueous solution) to 8.6.

After winding the hair on curlers the above permanent waving composition is uniformly applied onto the curlers. The permanent waving composition is left on the curlers for 10 minutes. An infrared drying hood is used at a temperature of 40°C. During the treating time a much better and more pleasant smell is notices by the hairdresser as well as by the customer compared to commonly used permanent waving compositions. The hair is then rinsed with lukewarm water. Thereafter 80g of the fixing composition of example 1 is applied onto the curler. The fixing composition is left on the hair for 4 minutes. Finally the hair is again rinsed with lukewarm water and the curlers are removed. The hair shows in dry and wet condition a well groomed look, a good touch and a fine wet combing property. The hair displays a good elasticity from the tips to the roots. Even after 10 days the hair keeps its pleasant smell in the dry or wet condition.

### Example 11 Low Alkaline Permanent Waving Composition for Colored Hair

| | |
|---|---|
| 7.20 g | N-(3'-hydroxypropyl)-2-mercaptoacetamide |
| 0.50 g | ammonia (25 % aqueous solution) |
| 2.00 g | ammonium hydrogen carbonate |
| 0.50 g | aminoethylaminopropylsiloxane/dimethylsiloxan-copolymer emulsion; Dow Corning 2-8566 ^{®} (CTFA: AMODIMETHICONE) |
| 0.60 g | CTFA: DIPALMITYLETHYL DIMETHYLAMMONIUMCHLORIDE |
| 1.50 g | 1,2-propylene glycol |
| 1.50 g | dipropyleneglycol monoethylether |
| 1.00 g | castor oil ethoxylated with 35 moles ethylenoxide (CTFA: PEG-35 CASTOR OIL) |
| 1.00 g | cocos oil ethoxylated with 10 moles ethylenoxide Genapol^{®} C 100 (CTFA: COCETH-10) |
| 0.30 g | styrene/vinyl pyrrolidone copolymer Antara^{®} 430 (CTFA: Styrene/VP Copolymer) |
| 0.50 g | perfume oil |
| ad 100.00 g | water |

The pH of the composition is adjusted with ammonia (25 % aqueous solution) to 8.2.

After winding the hair on curlers the above permanent waving composition is uniformly applied onto the curlers. The permanent waving composition is left on the curlers for 10 minutes. An infrared drying hood is used at a temperature of 38°C. During the treating time a much better and more pleasant smell is notices by the hairdresser as well as by the customer compared to permanent waving compositions commonly used. The hair is then rinsed with lukewarm water. Thereafter 80g of a fixing composition of example 1 is applied onto the curler.

The fixing composition is left on the hair for 5 minutes. Finally the hair is rinsed again with lukewarm water and the curlers are removed. The hair shows an improved soft feel of wet and dry hair, an improved uniformity of the curls and an improved elasticity of hair from the roots to the tips. Even after 5 days the hair keeps its pleasant smell either in dry or in wet condition. It displays a good fastness, even over a long period after the treatment, to humidity particularly sweat. During or after the hair treating procedure described above neither irritations of the skin nor other effects on the skin were realized.

### Example 12 Low Alkaline Permanent Waving Composition for Normal Hair

| | |
|---|---|
| 14.00 g | N-(3'-hydroxypropyl)-2-mercaptoacetamide (90 % by weight aqueous solution) |
| 2.00 g | sodium carbonate |
| 0.50 g | polydimethyldially ammonium chloride |
| 0.60 g | CTFA: BEHENTRIMONIUM METHOSULFAT |
| 1.00 g | isopropyl alcohol |
| 1.50 g | 1,2-propylenglycol |
| 2.00 g | dipropylenglycol monoethylether |
| 3.50 g | urea |
| 1.00 g | castor oil, ethoxylated with 35 moles ethylene oxide |
| | Chremophor^{®} EL (CTFA: PEG-35 CASTOR OIL) |
| ad 100.00 g | water |

The pH of this composition is adjusted with an aqueous solution of sodium carbonate (20 weight %) to 8.6.

After winding the hair on curlers the above permanent waving composition is uniformly applied onto the curlers. The permanent waving composition is left on the curlers for 10 minutes. An infrared drying hood is used at a temperature of 40°C. During the treating time a much better and more pleasant smell is notices by the hairdresser as well as by the customer compared to commonly used permanent waving compositions. The hair is than rinsed with lukewarm water. Thereafter 80g of the fixing composition of example 1 is applied onto the curler. The fixing composition is left on the hair for 4 minutes. Finally the hair is again rinsed with lukewarm water and the curlers are removed. The hair displays a good elasticity from the tips to the roots. Even after 10 days the hair keeps its pleasant smell in the dry or wet condition. It displays a good fastness, even over a long period after the treatment, to humidity particularly sweat.

### Example 13 Low Alkaline Permanent Waving for Unmanageable Hair

| | |
|---|---|
| 14.00 g | N-(3'-hydroxypropyl)-2-mercaptoacetamide (90% by weight aqueous solution) |
| 1.00 g | ethanolamine |
| 2.80 g | CTFA: CETRIMONIUM METHOSULFATE |
| 1.00 g | 1,3-butanediol |
| 1.50 g | 1,2-propylenglycol |
| 3.50 g | urea |
| 0.60 g | perfume oil |
| ad 100.00 g | water |

The pH of the composition is adjusted with ethanolamine to 8.9.

After winding the hair on curlers the above permanent waving composition is uniformly applied onto the curlers. An infrared drying hood is used at a temperature of 40°C. The permanent waving composition is left on the curlers for 14 minutes. During the treating time a much better and more pleasant smell is noticed by the hairdresser as well as by the customer compared to permanent waving compositions commonly used. The hair is than rinsed with lukewarm water. Thereafter 80g of a fixing composition of example 1 is applied onto the curler.

The fixing composition is left on the hair for 4 minutes. Finally the hair is rinsed again with lukewarm water and the curlers are removed. The thus-treated hair has a uniform, lively curliness. It displays a good fastness to humidity, particularly sweat, even over a long period after the treatment. Even after 10 days the hair keeps its pleasant smell either in dry or in wet condition. Not the slightest skin irritation was noticed.

### Example 14: Low Alkaline Permanent Waving Composition for Colored Hair

| | |
|---|---|
| 4.00 g | N-propyl-2-mercaptoacetamide |
| 3.00 g | cystein hydrochloride |
| 0.50 g | ammonia (25 % aqueous solution) |
| 1.50 g | ammonium hydrogen carbonate |
| 0.40 g | polydimethyldiallyl ammonium chloride |
| 0.90 g | ethanaminium-2-(hexadecyloxy)-N,N,N-trimethyl-2-oxo-chloride (CTFA: CETYL BETAINATE CHLORIDE) |
| 1.50 g | 1,2-propylenglycol |
| 2.00 g | dipropyleneglycol monoethyl ether |
| 2.00 g | urea |
| 1.00 g | hydrogenated castor oil, ethoxylated wit 40 moles of ethylene oxide (CTFA: PEG-40 HYDROGENATED CASTOR OIL) |
| 0.50 g | perfume oil |
| ad 100.00 g | water |

The pH of the composition is adjusted with ammonia (25 % aqueous solution) to 8.2

After winding the hair on curlers the above permanent waving composition is uniformly applied onto the curlers. The permanent waving composition is left on the curlers for 10 minutes. An infrared drying hood is used at a temperature of 40°C.

During the treating time a much better and more pleasant smell is notices by the hairdresser as well as by the customer compared to permanent waving compositions used commonly. The hair is than rinsed with lukewarm water. Thereafter 80g of a fixing composition of example 1 are applied onto the curler.

The fixing composition is left on the hair for 6 minutes. Finally the hair is again rinsed with lukewarm water and the curlers are removed. The hair displays a good fastness, even over a long period after the treatment, to humidity particularly sweat. It exhibits a good elasticity from the tips to the roots. Even after 5 days the hair keeps its pleasant smell either in dry or in wet condition. Not the slightest skin irritations were noticed.

### Example 15 Hair Straightening Composition for Dyed Hair

| | |
|---|---|
| 5.00 g | N-(3'-hydroxypropyl)-2-mercaptoacetamide (90 % by weight by weight aqueous solution) |
| 2.50 g | ammonium thioglycolate |
| 0.90 g | ammonia (25 % aqueous solution) |
| 9.50 g | cetylstearyl alcohol (Lanette^{®} O) |
| 2.50 g | cetylstearyl alcohol polyethylenglycol (25) ether (Cremophor^{®}A 25) |
| 2.50 g | Vaseline (CTFA: PETROLATUM) |
| 1.00 g | Dimethylsiloxane, 3-(3-((3-cocoamidopropyl)dimethylammonio)-2-hydroxyprpoxy)propyl groupterminated acetate; (Abil Quat^{®} 3474) (CTFA: QUATERNIUM-80; CAS 134737-05-6) |
| 0.80 g | CTFA: BEHENTRIMMONIUM CHLORIDE |
| 0.50 g | perfume oil |
| ad 100.00 g | water |

The pH of this composition is adjusted with ammonia (25% aqueous solution) to 8.2.

The above hair straightening cream is applied onto the dry hair by a brush. Thereafter the hair is combed several times to smoothen said hair. After the treating time of 20 minutes at a temperature of 25° C the hair is rinsed with water and than oxidatively post-treated with 90g of the fixing composition of example 1. After the treating time of 8 min the hair is rinsed again with water. Thereafter the hair is ironed with a hot iron. During the treating time a much better and more pleasant smell is notices by the hairdresser as well as by the customer compared to permanent straightening compositions commonly used. Even after 10 days the hair keeps its pleasant smell either in dry or in wet condition. The shaped hair displays a good fastness to humidity, particularly sweat, even over a long period after the treatment. Not the slightest skin irritation was noticed.

### Example 16 Hair Straightening Composition for Very Curly Hair

| | |
|---|---|
| 14.00 g | N-(3'-hydroxypropyl)-2-mercaptoacetamide (90 % by weight aqueous solution) |
| 3.00 g | monoethanolamine thioglycolate (70 % by weight aqueous solution) |
| 0.90 g | ethanolamine (85% by weight aqueous solution) |
| 11.00 g | cetylstearyl alcohol (Lanette^{®} O) |
| 2.50 g | cetylstearyl alcohol polyethylenglycol (25) ether (Cremophor^{®} A 25) |
| 0.30 g | CTFA: CETRIMMONIUM CHLORIDE |
| 3.20 g | Vaseline (CTFA: PETROLATUM) |
| 0.50 g | perfume oil |
| ad 100.00 g | water |

The pH of this composition is adjusted with ethanolamine (85% by weight aqueous solution) is 9.3

The above hair straightening cream is applied onto the dry hair with a brush. Thereafter the hair is combed several times to smoothen said hair. After the treating time of 16 minutes at a temperature of 28° C the hair is rinsed with water and than oxidatively post-treated with 80g of the fixing composition of example 1. After the treating time of 7 min the hair is rinsed again with water. Thereafter the hair is ironed with a hot iron. During the treating time a much better and more pleasant smell is notices by the hairdresser as well as by the customer compared to permanent straightening compositions commonly used. The shaped hair displays a good fastness to humidity, particularly sweat, even over a long period after the treatment. Not the slightest skin irritation was noticed.

### Example 17 Hair Straightening Composition for curly normal hair

| | |
|---|---|
| 14.00 g | N-(3'-hydroxypropyl)-2-mercaptoacetamide (90 % by weight aqueous solution) |
| 0.20 g | ammonia (25 % aqueous solution) |
| 3.50 g | ammonium hydrogen carbonate |
| 0.60 g | stearyl trimethyl ammonium chloride |
| 11.00 g | cetylstearyl alcohol (Lanette^{®} O) |
| 2.50 g | cetylstearyl alcohol polyethylenglycol (20) ether (Cremophor^{®} A 20) |
| 3.20 g | Vaseline (CTFA: PETROLATUM) |
| 0.50 g | perfume oil |
| ad 100.00 g | water |

The pH of this composition is adjusted with ammonia (25 % aqueous solution) to 9.0.

The above hair straightening cream is applied onto the dry hair with a brush. Thereafter the hair is combed several times to smoothen said hair. After the treating time of 16 minutes at a temperature of 28° C the hair is rinsed with water and than oxidatively post-treated with 80g of the fixing composition of example 1. After the treating time of 7 min the hair is rinsed again with water. Thereafter the hair is ironed with a hot iron. During the treating time a much better and more pleasant smell is noticed by the hairdresser as well as by the customer compared to commonly used permanent straightening compositions. The shaped hair displays a good fastness to humidity, particularly sweat, even over a long period after the treatment.

### Examples 18 to 21 Hair Straightening Compositions for Very Curly Hair

| **Example** | **18** | **19** | **20** | **21** |
|---|---|---|---|---|
| N-(3'-hydroxypropyl)-2-mercaptoacetamide (90 % by weight aqueous solution) | 14.00 g | 17.00 g | 15.00 g | 13.00 g |
| sodium carbonate | 0.60 g | 0.80 g | 0.70 g | 0.50 g |
| CTFA: DISTEARYL-DIMONIUM CHLORIDE | 0.50 g | 0.20 g | - - - | - - - |
| CTFA: BEHENOYL-PG-TRIMONIUM CHLORIDE | - - - | 0.30 g | - - - | - - - |
| CTFA: PEG-15 STEARMONIUM CHLORIDE | - - - | - - - | 0.90 g | - - - |
| CTFA: LAURTRIMONIUM CHLORIDE | - - - | - - - | - - - | 0.30 g |
| ammonia (25 % aqueous solution) | 0.50 g | 5.50 g | 3.50 g | 4.00 g |
| ammonium hydrogen carbonate | 2.50 g | - - - | - - - | - - - |
| cetylstearyl alcohol (Lanette^{®} O) | 11.00 g | 11.00 g | 11.00 g | 11.00 g |
| cetylstearyl alcohol polyethylenglycol (20) ether (Cremophor^{®} A 20) | 2.50 g | 2.50 g | - - - | - - - |
| cetylstearyl alcohol polyethylenglycol (20) ether (Cremophor^{®} A 25) | - - - | - - - | 2.50 g | 2.50 g |
| Vaseline (CTFA: | 3.20 g | 3.20 g | 3.20 g | 3.20 g |
| PETROLATUM) | | | | |
| perfume oil | 0.50 g | 0.50 g | 0.50 g | 0.50 g |
| water | ad 100.00 g | ad 100.00 g | ad 100.00 g | ad 100.00 g |

The pH is adjusted with sodium carbonate to 9.5.

The above hair straightening cream is applied onto the dry hair with a brush. Thereafter the hair is combed several times to smoothen said hair. After the treating time of 16 minutes at a temperature of 28°C the hair is rinsed with water and than oxidatively post-treated with 80g of the fixing composition of example 1. After the treating time of 7 min the hair is rinsed again with water. Thereafter the hair is ironed with a hot iron. During the treating time a much better and more pleasant smell is notices by the hairdresser as well as by the customer compared to permanent straightening compositions commonly used. The shaped hair displays a good fastness, even over a long period after the treatment, to humidity particularly sweat.

### Examples 22 to 25 Hair Straightening Compositions for Dyed Hair

| Example | 22 | 23 | 24 | 25 |
|---|---|---|---|---|
| N-(3'-hydroxypropyl)-2-mercaptoacetamide (90 % by weight aqueous solution) | 8.00 g | 9.00 g | 11.00 g | 11.00 g |
| cysteine hydrochloride | 2.00 g | 1.00 g | - | - |
| ammonia, 25% aqueous solution | 1.50 g | 2.00 g | - | - |
| ethanolamine | - | - | 0.70 g | 0.60 g |
| dicetyl dimethyl ammonium chloride | 1.90 g | | | |
| stearyl trimethyl ammonium chloride | - | 0.60 g | - | - |
| cetyl trimethyl ammonium phosphate | - | - | 0.50 g | - |
| behenyl trimethyl ammonium chloride | - | - | - | 1.20 g |
| cetylstearyl alcohol (Lanette^{®} O) | 11.00 g | 11.00 g | 11.00 g | 11.00 g |
| cetylstearyl alcohol polyethylenglycol (20) ether (Cremophor^{®} A 20) | 2.50 g | 2.50 g | 2.50 g | 2.50 g |
| Vaseline (CTFA: PETROLATUM) | 3.20 g | 3.20 g | 3.20 g | 3.20 g |
| perfume oil | 0.50 g | 0.50 g | 0.50 g | 0.50 g |
| water | ad 100.00 g | ad 100.00 g | ad 100.00 g | ad 100.00 g |

The pH of the examples 22 and 23 of this composition is adjusted with ammonia (25 % aqueous solution) to 8.3. The pH of the examples 24 and 25 is adjusted with ethanolamine (85 % aqueous solution) to 8.3

The above hair straightening cream is applied onto the dry hair with a brush. Thereafter the hair is combed several times to smoothen said hair. After the treating time of 16 minutes at a temperature of 28°C the hair is rinsed with water and than oxidatively post-treated with 80g of the fixing composition of example 1. After the treating time of 7 min the hair is rinsed again with water. During the treating time a much better and more pleasant smell is notices by the hairdresser as well as by the customer compared to permanent straightening compositions commonly used. The shaped hair displays a good fastness, even over a long period after the treatment, to humidity particularly sweat. The hair exhibits a uniform straight shape from the tips to the roots and the hair tips show a good structure. During or after the hair treating procedure described above neither irritations of the skin nor other effects on the skin were realized.

### Examples 26 to 29 Hair Straightening Compositions for Normal Hair

| Example | 26 | 27 | 28 | 29 |
|---|---|---|---|---|
| N-(3'-hydroxypropyl)-2-mercaptoacetamide (90 % by weight aqueous solution) | 11.00 g | 13.00 g | 12.00 g | 12.00 g |
| sodium carbonate | 0.50 g | 0.90 g | - | - |
| ethanolamine | - | - | 0.70 g | 0.60 g |
| cetyldimethyl hydroxyethyl ammonium phosphate | 1.90 g | | | |
| stearyltrimethylammonium chloride | - | 0.40 g | - | - |
| behentrimmonium methosulfate | - | - | 0.50 g | - |
| dipalmitylethyl dimethylammoniumchloride | - | - | - | 2.10 g |
| cetylstearyl alcohol (Lanette^{®} O) | 11.00 g | 11.00 g | 11.00 g | 11.00 g |
| cetylstearyl alcohol polyethylenglycol (20) ether (Cremophor^{®} A 20) | 2.50 g | 2.50 g | 2.50 g | 2.50 g |
| Vaseline (CTFA: PETROLATUM) | 3.20 g | 3.20 g | 3.20 g | 3.20 g |
| perfume oil | 0.50 g | 0.50 g | 0.50 g | 0.50 g |
| water | ad 100.00 g | ad 100.00 g | ad 100.00 g | ad 100.00 g |

The pH of the examples 26 and 27 of this composition is adjusted with sodium carbonate (20% aqueous solution) to 9.0. The pH of the examples 28 and 29 is adjusted with ethanolamine (85% aqueous solution) to 9.0.

The above hair straightening cream is applied onto the dry hair with a brush. Thereafter the hair is combed several times to smoothen said hair. After the treating time of 16 minutes at a temperature of 28°C the hair is rinsed with water and than oxidatively post-treated with 80g of the fixing composition of example 1. After the treating time of 7 min the hair is rinsed again with water. During the treating time a much better and more pleasant smell is notices by the hairdresser as well as by the customer compared to permanent straightening compositions commonly used. The shaped hair displays a good fastness, even over a long period after the treatment, to humidity particularly sweat. The hair exhibits a uniform straight shape from the tips to the roots and the hair tips show a good structure. During or after the hair treating procedure described above neither irritations of the skin nor other effects on the skin were realized.

## Claims

1. A composition for permanent hair shaping comprising
(i) 1% to 30 % by weight of an N-alkyl-2-mercaptoacetamide of formula 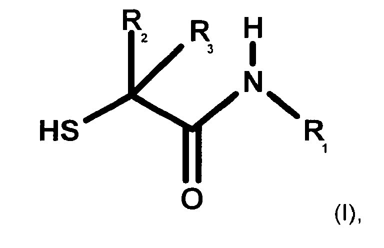 or the salt thereof, wherein R₁ is a straight chain alkyl residue with 3 to 6 carbon atoms or a straight chain hydroxyalkyl residue with 3 to 6 carbon atoms, R₂ and R₃ are, independently from each other, hydrogen or straight chain alkyl residues with 1 to 3 carbon atoms,
(ii) 0.01% to about 20% by weight of at least one at least one cationic surfactant that contains either a quaternary ammonium group or a protonable nitrogen group, and
(iii) 5% to 95% by weight of water.

2. A composition according to claim 1, wherein the N-alkyl-2-mercapto acetamide of formula (I) is selected from N-propyl-2-mercaptoacetamide, N-(2'-hydroxypropyl)-2-mercaptoacetamide and N-(3'-hydroxypropyl)-2-mercaptoacetamide.

3. A composition according to claim 1 or 2, wherein the N-alkyl-2-mercapto acetamide of formula (I) is N-(3'-hydroxypropyl)-2-mercaptoacetamide.

4. A composition according to anyone of claims 1 or 3, wherein the N-alkyl-2-mercaptoacetamide of formula (I) is present in an amount of from 5 to 20 % by weight.

5. A composition as recited in any one of claims 1 to 4, wherein the total amount of the hair keratin-reducing agents in the permanent shaping composition is from 5% to 20% by weight.

6. A composition as recited in any one of claims 1 to 5, wherein the cationic surfactant is selected from the group consisting of cetyl trimethyl ammonium phosphate, behenyl trimethylammonium chloride, behenyl trimethylammonium methosulfate, stearyl trimethylammonium chloride, octadecyl dimethyl benzyl ammonium chloride, docosyl dimethyl benzyl ammonium chloride, dicetyl dimethyl ammonium chloride, cetyl trimethyl ammonium chloride, cetyl trimethyl ammonium bromide, ditallow dimethyl ammonium chloride; stearyl amidopropyl dimethylamine, esterquats, diesterquats, dipalmitylethyl dimethylammoniumchloride, a mixture of distearoylethyl hydroxyethyl ammonium methosulfate and cetylstearyl alcohol, the salts of the amines dimethylaminopropyl stearamid, stearyl dimethylamin oxide, dimethylaminopropyl lauramide, N,N-dimethyl-1-behenamine-N-oxide, N-[3-(dimethylamino)propyl]-cocoamide, diethylaminoethyl stearamide, N,N-dimethyl-1-octadecanamine, soyamine, soy dimethylamine, myristylamine, tridecylamine, ethylstearylamine, N-tallowpropane diamine, ethoxylated (5 moles EO) stearylamine and N,N-bis(2-hydroxyethyl)-stearylamine; distearyl dimethylammonium chloride, octadecylnitrilio)tri-1,2-ethanediyl)tris hydroxyphosphate, polyethylenglycol(5)stearyl ammoniumlactate, N-eicosyl-1-docosanamine, 3-behenoyloxy-2-hydroxypropyl-trimethylammonium chloride, trimethylaminopropyllaureate, coconut trimethylammonium chloride, benzyl trimethylammonium chloride, N,N,N',N',N'-pentamethyl-N-tallowalkyl-1,3-propane-diammonium dichloride, lauryl trimethylammonium chloride, cetyl dimethyl hydroxyethyl ammonium phosphate, polyethyleneglycol (15) stearmonium chloride, N-[3-(dimethylamino)propyl]-tetradecanamide phosphate, N-(stearoyl colamino formylmethyl)-pyridinium chloride, 1,3-bis[(N,N-dimethyl-N-stearyl)]-2-hydroxy-propane dichloride, dicocos dimethyl ammonium chloride, hydroxycetyl hydroxyethyl dimethyl ammonium chloride, (2-ethyl-hexyl)-dimethyl-stearyl-ammonium chloride, tricetyl monomethyl ammonium chloride, 2-hydroxypropanoic acid compd. with N-(3-(dimethylamino)propyl)-octadecanamide, 3-trimethylamino-2-hydroxy-propyl isostearate chloride, 3-trimethylamino-2-hydroxy-propyl palmitate chloride, trimethyl-[(3-octadecyloxy)propyl]ammonium chloride, dioleoylethyl hydroxyethyl ammonium methosulfate, stearylamine hydrochloride, soyamine chloride, and citric acid compd. with N-(3-(dimethylamino)propyl)-octadecanamide.

7. A composition as recited in any one of claims 1 to 8, wherein the cationic surfactant is selected from the group consisting of cetyl trimethyl ammonium phosphate, behenyl trimethyl ammonium chloride, behenyl trimethyl ammonium methosulfate, stearyl trimethyl ammonium chloride, octadecyl dimethyl benzyl ammonium chloride, docosyl dimethyl benzyl ammonium chloride, dicetyl dimethyl ammonium chloride, cetyl trimethyl ammonium chloride, cetyl trimethyl ammonium bromide, ditallow dimethyl ammonium chloride; stearyl amidopropyl dimethylamine, esterquats, diesterquats, dipalmitylethyl dimethylammoniumchloride and a mixture of distearoylethyl hydroxyethyl ammonium methosulfate with cetylstearyl alcohol.

8. A composition as recited in claim 1, wherein quaternary ammonium group of the cationic surfactant has the general formula (II)
[NR₄,R₅,R₆,R₇]⁺· X⁻
wherein R₄ to R₇ are independent from each other a saturated or unsaturated aliphatic group of from 1 to 26 carbon atoms or an alkoxy-, polyoxyalkylene-, alkylamido-, hydroxyalkyl-, aminoalkyl-, phenyl-, alkylphenyl-, alkylpyridyl- or benzyl group of from 1 to 26 carbon atoms in the alkyl or alkylene residue; and X⁻ is a salt-forming anion selected from the group consisting of chloride, bromide, iodide; acetate, citrate, lactate, glycolate, phosphate, nitrate, sulfate and methylsulfate.

9. A composition as recited in claim 8, wherein in the formula (II) of the cationic surfactant one or two of the residues R₄ to R₇ are saturated or unsaturated aliphatic hydrocarbon groups of 12 to 26 carbon atoms and the other residues are the independently from each other -CH₃ or -C₂H₃ groups.

10. A composition as recited in claim 8 or 9, wherein in the formula (II) of the cationic surfactant one or two of the residues R₄ to R₇ are saturated or unsaturated aliphatic hydrocarbon groups of 16 to 22 carbon atoms and the other residues are independently from each other -CH₃ or -C₂H₃ groups.

11. A composition as recited in any one of claims 1 to 10, wherein the total amount of the cationic surfactant of is from 0.05% to 8% by weight.

12. Composition as recited in any one of claims 1 to 11, wherein the permanent shaping composition comprises a disulfide of a hair keratin-reducing agent.

13. A composition as recited in claim 12, wherein the disulfide of the hair keratin-reducing agent is selected from the group consisting of 2,2'-dithio-bis[N-(3-hydroxypropyl)-acetamide], 2,2'-dithio-bis[N-(propyl)-acetamide], 2,2'-dithio-bis[N-(2-hydroxypropyl)-acetamide] and dithioglycolic acid or the salt thereof.

14. A composition as recited in one of claims 12 or 13, wherein the disulfide of the hair keratin-reducing agent is contained in a quantity of from 0.5% to 15% by weight.

15. A composition as recited in any one of claims 12 to 14, wherein the ratio of the hair keratin-reducing agent or the mixture thereof to the disulfide of a hair keratin-reducing agent is from 20 : 1 to 1 : 2.

16. A composition according to anyone of claims 1 to 15, wherein the pH-value is from 2 to 10.

17. A composition according to anyone of claims 1 to 15, wherein the pH-value is from 4 to 9.5.

18. A composition according to anyone of claims 1 to 17, wherein it comprises at least one cationic polymer, selected from the group consisting of: polymer of dimethyl diallyl ammonium chloride; vinylpyrrolidon/dimethylaminoethylmethacrylat methosulfate; aminoethylaminopropylsiloxane/dimethylsiloxan-copolymer emulsion, quaternized vinylpyrrolidon/dimethylaminoethylmethacrylate-copolymer, copolymer of methylvinylimidazoliumchloride and vinylpyrrolidone, the quaternary ammonium salt of the terpolymer of acrylic/diallyldimethylammonium chloride/acrylamide; ethanaminium, N,N,N-trimethyl-2-[(methyl-1-oxo-2-propenyl)oxy]-chloride homopolymer; the copolymer of hydroxyethyl cellulose and diallyldimethylammonium chloride, the copolymer of acrylamide and diallyldimethylammonium chloride; the copolymer of acrylamide and betamethacrylyloxyethyl trimethyl ammonium chloride; the copolymer of methacrylamide and betamethacrylyloxyethyl trimethyl ammonium chloride; the copolymer of polyvinyl pyrrolidone and imidazolimine methochloride, the copolymer of diallyldimethyl ammonium chloride and acrylic acid, the copolymer of vinyl pyrrolidone and methacrylamidopropyl trimethyl ammonium chloride, the methosulfate of the copolymer of methacryloyloxyethyl trimethylammonium and methacryloyloxyethyl dimethylacetylammonium, quaternized hydroxyethyl cellulose; copolymer of hydroxyethyl cellulose and diallyldimethylammonium chloride; dimethylsiloxane 3-(3-((3-cocoamidopropyl)dimethylammonio)-2-hydroxy-prpoxy)propyl groupterminated acetate; dimethylsiloxane, 3-(3-((3-cocoamidopropyl)-dimethylammonio)-2-hydroxyprpoxy)propyl groupterminated acetate; N-(3-chloroallyl)hexaminium chloride; the polyethylene glycol derivative of aminoethylaminopropylsiloxane/dimethylsiloxan-copolymer and cationic silicone polymers.

19. A composition as recited in claims 17, wherein the total amount of the cationic polymer is from 0.1 % to 6% by weight.

20. A method for permanent shaping of hair, said method comprising the steps of:
(a) putting the hair in a desired shape;
(b) before and/or after the hair is put in the desired shape, applying an amount of a permanent shaping composition to the hair and allowing the permanent shaping composition to act on the hair for a predetermined acting time sufficient for the permanent shaping of the hair;
(c) subsequently rinsing the hair with water or applying an intermediate treatment agent,
(d) performing an oxidative post-treatment of the hair;
(e) after the oxidative post-treatment, rinsing the hair with water and drying the hair, wherein said permanent shaping composition is a composition according to anyone of claims 1 to 18.

21. Method as recited in claim 20, wherein the intermediate treatment composition comprises at least one member of betaine, citric acid, lactic acid, and/or glyoxylic acid.

22. Method as recited in one of claims 20 or 21, wherein the intermediate treatment composition has a pH of from 2 to 4.

23. Method as recited in one of claims 20 to 22, wherein the intermediate treatment composition has an action period of from 3 to 5 minutes.

24. The method recited in any one of claims 20 to 23, further comprising allowing said composition for permanent shaping of hair to act on the hair for from 5 to 20 minutes prior to said rinsing of the hair with water.

25. The method recited in any one of claims 20 to 24, wherein said applied amount of said permanent shaping composition is from 60 g to 120 g.

26. The method as defined in claim 25, further comprising putting the hair in a water wave after having oxidatively post-treated the hair.
